(19) Europäisches Patentamt
European Patent Office
Office européen des brevets

(11) EP 1 437 401 B1

(12) **EUROPÄISCHE PATENTSCHRIFT**

(45) Veröffentlichungstag und Bekanntmachung des Hinweises auf die Patenterteilung:
**15.12.2010 Patentblatt 2010/50**

(51) Int Cl.:
*C12N 9/02* (2006.01) *C12P 7/42* (2006.01)

(21) Anmeldenummer: **04000120.8**

(22) Anmeldetag: **07.01.2004**

(54) **Oxidoreduktase**

Oxidoreductase

Oxidoreductase

(84) Benannte Vertragsstaaten:
**AT BE BG CH CY CZ DE DK EE ES FI FR GB GR HU IE IT LI LU MC NL PT RO SE SI SK TR**

(30) Priorität: **09.01.2003 DE 10300335**

(43) Veröffentlichungstag der Anmeldung:
**14.07.2004 Patentblatt 2004/29**

(73) Patentinhaber: **IEP GmbH**
**65203 Wiesbaden (DE)**

(72) Erfinder:
- **Bobkova, Maria**
  **65510 Idstein (DE)**
- **Gupta, Antje, Dr.**
  **65207 Wiesbaden (DE)**
- **Zimmer, Anke**
  **65205 Wiesbaden (DE)**

(74) Vertreter: **Schwarz, Albin et al**
**Schwarz & Partner**
**Patentanwälte**
**Wipplingerstraße 30**
**1010 Wien (AT)**

(56) Entgegenhaltungen:
**EP-A- 0 796 914 WO-A-96/01892**
**WO-A-02/086126**

- **DATABASE EBI [Online] retrieved from EMBL Database accession no. ABP28195 XP002275433 & WO 02 34771 A (CHIRON SPA) 2. Mai 2002 (2002-05-02)**

EP 1 437 401 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft eine Oxidoreduktase, eine isolierte DNA-Sequenz der Oxidoreduktase, ein Fusionsprotein auf der Basis der Oxidoreduktase und ein Verfahren zur enantioselektiven Gewinnung von S-2-Hydroxysäureestern.

2-Hydroxysäuren und deren Ester stellen wichtige chirale Synthesegrundbausteine dar aus denen eine Reihe von Verbindungen unter Erhalt der Chiralität am C2-Atom gewonnen werden können, beispielsweise Epoxide, Alkylester, Hydrazinylester, alpha-N-Alkoxyaminoester oder alpha-Aminoester.

Zahlreiche Forschungsarbeiten wurden bisher der Entwicklung von Methoden zur Darstellung enantiomerenreiner 2-Hydroxysäuren und deren Ester gewidmet, wobei verschiedene chemische und biokatalytische Ansätze betrachtet wurden. Bisher ist es noch nicht gelungen Verfahren zu entwickeln welche den Anforderungen der Produktion im industriellen Maßstab genügen. Insbesondere bei der Herstellung von 2-Hydroxysäuren und ihren Estern erscheint die enzymkatalysierte Einführung des chiralen Zentrum der Synthese durch chemischen Katalysatoren überlegen.

Bei den enzymkatalysierten Verfahren gibt es momentan drei verschiedene Verfahren. Ein Weg ist die oxynitrilase-Katalysierte Synthese chiraler Cyanohydrine und deren anschließender, oft ebenfalls enzymkatalysierter Hydrolyse (Biotransformations in Organic Chemistry, A Textbook. 4th Edition, Springer (2000), K. Faber; Cyanhydrinformation). Nachteil dieses Verfahrens ist die Verwendung des toxischen HCN.

Ein weiteres Verfahren ist die Racematspaltung von 2-Hydroxysäureestern mit Hilfe von Lipasen, beispielsweise aus Pseudomonas flourescens (J. Org. Chem. 55, 812-815 (1991), Kinetic Resolution of 2-substituted Esters catalysed by a Lipase Ex. Pseudomonas floureszens, Kalaritis, P. et al.). Nachteil dieser Methode ist die theoretische Ausbeute von nur 50 %.

Ein weiteres Verfahren ist die Synthese chiraler 2-Hydroxysäuren und deren Ester durch Reduktion prochiraler 2-Oxosäuren oder deren Ester. Bekannt ist die Transformationen mit ganzen Hefezellen oder Zellen von Proteus vulgaris oder Proteus mirabilis oder Verfahren mit isolierten Enzymen. Bei der Transformationen mit ganzen Hefezellen wurde die reduktive Enzymaktivität an 2-Oxosäuren auf die Enzyme Lactat-Dehydrogenase oder Malat-Dehydrogenase zurückgeführt (Ramesh N. Patel Stereoselective Biocatalyse, NY (2000), 14. Stereoselective Synthesis of Chiral Compounds Using Whole- Cell Biocatalysis, Paola D'Arrigo, Giuseppe Pedrocchi-Fantoni and Stefano Servi), während bei den mit Proteus durchgeführten Reduktionen ein membranständiges Molybdän-abhängiges Eisen-Schwefelprotein für die Reaktion verantwortlich sein soll (Eur J Biochem (1994); 222(3):1025-32 ,The (2R)-hydroxycarboxylate-viologen-oxidoreductase from Proteus vulgaris is a molybdenum-containing iron-sulphur protein, Trautwein T, Krauss F, Lottspeich F, Simon H.).

In bekannten Verfahren zur Reduktion von 2-Oxosäureestern werden die isolierten Enzymen (D/L)- Lactatdehydrogenase (US 5,686,275), (D/L)-Dihydroxyisocaproat-Dehydrogenase (US 6,033,882) oder D-Mandelatedehydrogenase (Appl Environ Microbiol 2002 Feb;68(2):947-51, Two forms of NAD-dependent D-mandelate dehydrogenase in Enterococcus faecalis IAM 10071. Tamura Y. et al 10) eingesetzt, die auch kloniert, überexprimiert und kommerziell erhältlich sind. Diese Enzyme sind NADH abhängig und setzen 2-Oxosäureester nicht um. Ferner ist bekannt, dass Enzyme, die 2-Oxosäuren oder deren Ester reduzieren, sekundären Alkohole nicht umsetzen.

Ferner sind Verfahren bekannt, worin das Coenzym NAD mit Formiat-Dehydrogenase, beispielsweise aus Candida boidinii oder auch rekombinant aus Pseudomonas floureszens regeneriert wird (Biotechnology , Biotransformations I (Rehm and Reed) 9. Alcohol Dehydrogenases-Characteristics, Design of Reaction Conditions J. Peters, WILEY-VCH -Verlag, (1998)). Beispielhaft ist hier das Verfahren zur enzymatischen Herstellung von R-2-Hydroxy-4-phenylbuttersäure mit Hilfe von D-Lactat-Dehydrogenase aus Staphylococcus epidermidis genannt (Industrial Biotransformations, Liese, K. Seelbach, C.Wandrey, WILEY-VCH -Verlag, (2000)). Nachteil der Coenzymregenerierung mit Formiat-Dehydrogenase ist die geringe spezifische Aktivität der Formiat-Dehydrogenase (4 bis 10 U/mg) und die hohen Kosten zur Herstellung des Enzyms. Dadurch ist es aus ökonomischer Sicht notwendig das Enzym mehrfach zu verwenden, was in einer vergleichsweise wesentlich komplizierteren und somit teureren Prozessführung resultiert.

Aufgabe der Erfindung ist es, durch eine Oxidoreduktase die genannten Nachteile der Verfahren aus dem Stand der Technik zu beheben.

Diese Aufgabe wird erfindungsgemäß durch eine Oxidoreduktase gelöst, welche 2-Oxosäureester in Gegenwart von NADPH und Wasser zu den entsprechenden S-2-Hydroxysäureestern reduziert, und dadurch gekennzeichnet ist, dass sie mehr als 70 % Identität mit der Aminosäuresequenz SEQ ID NO: 18 und eine spezifische Aktivität von mehr als 1 $\mu$mol pro mg aufweist, bezogen auf die Umsetzung von Ethyl-2-oxo-4-phenyl- butyrat zu S-Ethyt-2-hydroxy-4-phenytbutyrat aufweist.

Die Erfindung betrifft unter anderem Oxidoreduktasen, die beispielsweise aus Lactobacillus (L.) reuteri, L. kefiri, L. kandleri, L. parabuchneri, L. cellobiosus oder L. fermentum gewonnen werden können.

Die Erfindung betrifft ferner die Oxidoreduktase aus Lactobacillus reuteri, die die Aminosäuresequenz gemäß SEQ ID NO: 18, wie im anliegenden Sequenzprotokoll beschrieben, aufweist.

Bevorzugt sind Oxidoreduktasen, die 80 % bis 99,5 %, insbesondere 90 % bis 99,5 %, insbesondere bevorzugt 99 % bis 99,5 % Identität mit der Aminosäuresequenz von SEQ ID NO: 18 aufweisen. Die Messung der spezifischen Aktivität der Oxidoreduktase gemäß SEQ ID NO: 18 oder ihrer Derivate oder Analogons erfolgt mit dem Testsystem, das in Beispiel 2 beschrieben wird.

Die Erfindung betrifft ferner eine Oxidoreduktase, die dadurch gekennzeichnet ist, dass sie 1 bis 50 Aminosäuren zusätzlich oder 1 bis 50 Aminosäuren weniger aufweist als die Oxidoreduktase mit der Aminosäuresequenz SEQ ID NO: 18. Bevorzugt sind Oxidoreduktasen, worin 1 bis 25 Aminosäuren, insbesondere 2 bis 20 Aminosäuren, bevorzugt 3 bis 10 Aminosäuren mehr oder weniger in der Aminosäuresequenz von SEQ ID NO: 18 vorkommen.

Die Erfindung betrifft ferner eine Oxidoreduktase, die dadurch gekennzeichnet ist, dass sie die Aminosäuresequenz von SEQ ID NO: 18 aufweist und ein-, zwei-, drei-, vier- oder fünffach durch ein wasserlösliches Polymer modifiziert ist. Ein Beispiel für ein wasserlösliches Polymer ist Polyethylenglycol. Die Bindung des Polyethylenglycols erfolgt bevorzugt am N-terminalen Ende der Oxidoreduktase gemäß SEQ ID NO: 18. Die Oxidoreduktase gemäß SEQ ID NO: 18 kann auch an einen Festkörper wie Polyethylen, Polystyrol, Polysaccharid, Cellulose oder Cellulosederivat gebunden sein.

Die Erfindung betrifft ferner ein Fusionsprotein, das dadurch gekennzeichnet ist, dass es die Oxidoreduktase mit der Aminosäuresequenz SEQ ID NO: 18 darstellt, und dass die Oxidoreduktase mit einem weiteren Polypeptid am N-terminalen oder Carboxyterminalen Ende mit einer Peptidbindung verbunden ist. Fusionsproteine können beispielsweise leichter von anderen Proteinen abgetrennt werden oder werden in den Zellen in größeren Mengen exprimiert.

Die Erfindung betrifft ferner einen Antikörper, der spezifisch an die Oxidoreduktase gemäß SEQ ID NO: 18 bindet. Die Herstellung dieser Antikörper erfolgt nach bekannten Methoden durch Immunisierung von geeigneten Säugetieren wie Pferd, Maus, Ratte oder Schwein und anschließender Gewinnung der Antikörper. Die Antikörper können monoklonal oder polyklonal sein.

Die Erfindung betrifft auch eine isolierte Nukleinsäuresequenz, die für die Oxidoreduktase gemäß SEQ ID NO: 18 kodiert.

Die Erfindung betrifft ferner eine isolierte Desoxyribonukleinsäuresequenz (DNA-Sequenz) der Oxidoreduktase, die die Reduktion von 2-Oxosäureestern in Anwesenheit von NADPH und Wasser zu entsprechenden S-2-Hydroxysäureestern katalysiert, wobei die DNA-Sequenz ausgewählt ist aus der Gruppe, bestehend aus

a) SEQ ID NO: 19 oder ihrem komplementären Strang,
b) einer DNA-Sequenz, welche mit der DNA-Sequenz gemäß a) oder ihremkomplementären Strang hybridisiert, wobei die Hybridisierung unter stringenten Bedingungen erfolgt, und
c) einer DNA-Sequenz, welche auf Grund der Degeneration des genetischen Codes ein Protein kodiert, das durch eine oder mehrere der DNA-Sequenzen gemäß a) oder b) kodiert ist.

Die Hybridisierung ist beispielsweise von Sambrok and Russel in Molecular Cloning a laboratory Manual , Band 1, Kapitel 1, Protokoll 30-32, beschrieben.

Die Erfindung betrifft ferner eine isolierte DNA-Sequenz, die dadurch gekennzeichnet ist, dass sie mehr als 70 % Identität mit der DNA-Sequenz SEQ ID NO: 19 oder deren komplementären Strang aufweist und ein Protein kodiert, das eine spezifische Aktivität von mehr als 1 μmol pro mg aufweist, bezogen auf die Umsetzung von Ethyl-2-oxo-4-phenyl-butyrat zu S-Ethyl-2-hydroxy-4-phenylbutyrat. Bevorzugt sind DNA-Sequenzen, die 80 % bis 99,5 %, insbesondere 90 % bis 99,5 %, bevorzugt 99 % bis 99,5 % Identität mit der DNA-Sequenz SEQ ID NO: 19 aufweisen.

Die Erfindung betrifft ferner einen Klonierungsvektor, enthaltend eine oder mehrere der obengenannten Nukleinsäure- oder DNA-Sequenzen. Die Erfindung betrifft ferner einen Expressionsvektor, der sich in einer Bakterien-, Hefe-, Insekten-, Pflanzen- oder Säugetierzelle befindet und eine oder mehrere der obengenannten Nukleinsäure- oder DNA-Sequenzen enthält, und in geeigneter Weise mit einer Expressionskontrollsequenz verbunden ist.

Die Erfindung betrifft ferner eine Wirtszelle, die eine Bakterien-, Hefe-, Insekten-, Pflanzen- oder Säugetierzelle ist und mit einem der obengenannten Expressionsvektoren transformiert oder transfektiert wurde.

Die Identitäten der vorgenannten DNA-Sequenzen oder Aminosäuresequenz werden dadurch berechnet, dass man die Anzahl der Aminosäuren oder Nukleinsäurebasen summiert, die mit Teilsequenzen der jeweiligen Proteine oder DNA-Sequenzen identisch sind, durch die Gesamtzahl der Aminosäuren oder Nukleinsäurebasen dividiert und mit Hundert multipliziert.

Geeignete Klonierungsvektoren sind beispielsweise ppCR-Script, pCMV-Script , pBluescript (Stratagene), pDrive cloning Vector (Qiagen), pS Blue, pET Blue, pET LIC-Vektoren (Novagen) sowie TA-PCR Klonierungsvektoren (Invitrogene).

Geeignete Expressionsvektoren sind beispielsweise pKK223-3, pTrc99a, pUC, pTZ, pSK, pBluescript, pGEM, pQE, pET, PHUB, pPLc, pKC30, pRM1/pRM9, pTrxFus,pAS1, pGEx, pMAL, pTrx).

Geeignete Expressionskontrollsequenzen sind beispielsweise trp-lac (tac)-Promotor, trp-lac (trc) promotor, lac-Promotor, T7-Promotor, λpL-Promotor.

Die Oxidoreduktase aus Lactobacillus reuteri ist ein Homodimer mit einem Molekulargewicht bestimmt im SDS-Gel von 30 bis 35 kDa und einem Molekulargewicht bestimmt mit Gelpermeationchromatographie von 60 bis 65 kDa. Das Temperaturoptimum liegt im Bereich von 55 °C bis 60 °C und es hat ein pH-Optimum von 6,5 bis 7,0. Die Oxidoreduktase aus Lactobacillus reuteri weist eine gute Temperatur- und pH-Stabilität auf und ist im pH-Bereich von 4,5 bis 8,5 und im Temperaturbereich von 15 °C bis 50 °C für mindestens 5 Stunden stabil und zeigt ferner eine hohe Stabilität in organischen Lösungsmitteln.

Das Enzym ist insbesondere aus Mikroorganismen der Gattung Lactobacillus isolierbar und kann im spektrophotometrischen Test über die Abnahme von NADPH bei 340 nm in Gegenwart eines entsprechenden Substrates, beispielsweise Ethyl-2-oxo-4-phenylbuttersäure oder Ethyl-2-oxovaleriansäure, nachgewiesen werden.

Die erfindungsgemäße Oxidoreduktase aus Lactobacillus reuteri wurde kloniert und konnte in Escherichia (E.) coli mit Aktivitäten von 10 000 U/g bis 30 000 U/g E. coli Feuchtgewicht überexprimiert werden. Das Enzym ist somit preiswert und in großen Mengen verfügbar. In Datenbanken wurden keine verwandten Sequenzen gefunden, nur eine entfernte Verwandtschaft zu Enzymen der Gruppe Hydroxyacyl-CoA-Dehydrogenasen könnte vermutet werden.

Bei einem Verfahren zur Gewinnung der Oxidoreduktase aus Lactobacillus reuteri, wird die DNA, die für die Oxidoreduktase aus Lactobacillus reuteri kodiert, in einem geeigneten prokaryotischen oder eukaryotischen Mikroorganismus exprimiert. Bevorzugt wird die Oxidoreduktase aus Lactobacillus reuteri in einen Escherichia coli Stamm transformiert und exprimiert, insbesondere in Escherichia coli BL21star (DE3) Zellen (Invitrogen, cat. No. C6010-03, von E.coli BL21 abgeleitet, mit chromosomaler Kopie des T7 RNA - Polymerasegen unter Kontrolle des lacUV5-Promotor, ohne ompT und Lon-Protease, B121 star hat Mutation in RNaseE (rne131).

Die Oxidoreduktase aus Lactobacillus reuteri lässt sich beispielsweise so gewinnen, dass die rekombinanten Escherichia coli Zellen kultiviert werden, die Expression der Oxidoreduktase induziert wird und anschließend nach etwa 10 bis 18 Stunden (h) die Zellen durch Ultraschallbehandlung oder durch Naßvermahlung mit Glasperlen in einer Kugelmühle (Retsch, 10 min, 24 Hz) aufgeschlossen werden. Der erhaltene Zellextrakt kann entweder direkt verwendet werden oder weiter gereinigt werden. Dazu wird der Zellextrakt beispielsweise zentrifugiert und der erhaltene Überstand wird einer hydrophober Interaktionschromatographie unterworfen, beispielsweise hydrophober Interaktionschromatographie an Butylsepharose Fast Flow (Pharmacia) und anschließender Gelpermeation (Superdex 200 HR, Pharmacia).

Die Erfindung betrifft ferner ein Verfahren zur enantioselektiven Gewinnung von S-2-Hydroxysäureester, das dadurch gekennzeichnet ist, dass man 2-Oxosäureester in Anwesenheit von Oxidoreduktase, NADPH und Wasser zum entsprechenden S-2-Hydroxysäureester reduziert und den gebildeten S-2-Hydroxysäureester isoliert. Das erfindungsgemäße Verfahren weist eine hohe Standzeit auf, eine enantiomeren Reinheit von mehr als 94 % der hergestellten chiralen S-2-Hydroxysäureester und eine hohe Ausbeute bezogen auf die eingesetzte Menge des 2-Oxosäureesters.

Unter dem Begriff "NADPH" wird reduziertes Nicotinamid-adenin-dinucleotidphosphat verstanden. Unter dem Begriff "NADP" wird Nicotinamid-adenindinucleotid-phosphat verstanden.

Unter dem Begriff "2-Oxosäureestee' werden beispielsweise Verbindungen der Formel I

R2-C(O)-C(O)-O-R1 (I)

verstanden.

R1 steht für

1. -($C_1$-$C_{20}$)-Alkyl, worin Alkyl geradkettig oder verzweigt ist,
2. -($C_2$-$C_{20}$)-Alkenyl, worin Alkenyl geradkettig oder verzweigt ist und je nach Kettenlänge ein, zwei, drei oder vier Doppelbindungen enthält,
3. -($C_2$-$C_{20}$)-Alkinyl, worin Alkinyl geradkettig oder verzweigt ist und gegebenenfalls ein, zwei, drei oder vier Dreifachbindungen enthält,
4. -($C_6$-$C_{14}$)-Aryl,
5. -($C_1$-$C_8$)-Alkyl-($C_6$-$C_{14}$)-Aryl,
6. -($C_5$-$C_{14}$)-Heterocyclus, der unsubstituiert oder ein- bis dreifach substituiert ist durch Halogen, Hydroxyl, Amino oder Nitro, oder
7. -($C_3$-$C_7$)-Cycloalkyl,

R2 steht für

1. -($C_1$-$C_{20}$)-Alkyl, worin Alkyl geradkettig oder verzweigt ist,
2. -($C_2$-$C_{20}$)-Alkenyl, worin Alkenyl geradkettig oder verzweigt ist und je nach Kettenlänge ein, zwei, drei oder vier Doppelbindungen enthält,
3. -($C_2$-$C_{20}$)-Alkinyl, worin Alkinyl geradkettig oder verzweigt ist und gegebenenfalls ein, zwei, drei oder vier Dreifachbindungen enthält,
4. -($C_6$-$C_{14}$)-Aryl,

5. -($C_1$-$C_8$)-Alkyl-($C_6$-$C_{14}$)-Aryl,
6. -($C_5$-$C_{14}$)-Heterocyclus, der unsubstituiert oder ein- bis dreifach substituiert ist durch Halogen, Hydroxyl, Amino oder Nitro, oder
7. -($C_3$-$C_7$)-Cycloalkyl,
wobei die oben unter 1. bis 7. genannten Reste unsubstituiert sind oder ein- bis dreifach substituiert sind unabhängig voneinander durch

a) -OH,
b) Halogen, wie Fluor, Chlor, Brom oder Jod,
c) -$NO_2$ ,
d) -C(O)-O-($C_1$-$C_{20}$)-Alkyl, worin Alkyl gerade oder verzweigt ist und unsubstituiert oder ein- bis dreifach substituiert ist durch Halogen, Hydroxyl, Amino oder Nitro, oder
e) -($C_5$-$C_{14}$)-Heterocyclus, der unsubstituiert oder ein- bis dreifach substituiert ist durch Halogen, Hydroxyl, Amino oder Nitro.

Unter dem Begriff "S-2-Hydroxysäureester" sind Verbindungen der Formel II

R2-C(OH)-C(O)-O-R1 (II)

zu verstehen, wobei die -OH Gruppe in S-Konfiguration zum Kohlenstoffatom steht, an das sie gebunden ist und R1 und R2 die Bedeutung wie in Formel haben.

Unter dem Begriff Aryl werden aromatische Kohlenstoffreste verstanden mit 6 bis 14 Kohlenstoffatomen im Ring. -($C_6$-$C_{14}$)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter dem Begriff "-($C_1$-$C_{20}$)-Alkyl wird ein Kohlenwasserstoffrest verstanden, dessen Kohlenstoffkette geradkettig oder verzweigt ist und 1 bis 20 Kohlenstoffatome enthält beispielsweise Methyl, Ethyl, Propyl, Isopropyl, Butyl, tertiär-Butyl, Pentyl, Hexyl, Heptyl, Octyl, Nonenyl oder Decanyl.

Unter dem Begriff "-($C_3$-$C_7$)-Cycloalkyl" werden cyclische Kohlenwasserstoffreste verstanden wie Cyclopropyl, Cylobutyl, Cyclopentyl, Cyclohexyl oder Cycloheptyl.

Der Begriff "-($C_5$-$C_{14}$)-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, O und S. Beispiele für die Begriffe -($C_5$-$C_{14}$)-Heterocyclus sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolone, Oxadiazolidindione, Triazole, welche durch F, -CN, -$CF_3$ oder -C(O)-O-($C_1$-$C_4$)-Alkyl substituiert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl oder Dihydropyridinyl, Pyrrolidinyl, zum Beispiel 2-oder 3-(N-Methylpyrrolidinyl), Piperazinyl, Morpholinyl, Thiomorpholinyl, Tetrahydrothienyl oder Benzodioxolanyl.

Bevorzugte Verbindungen der Formel I sind beispielsweise Ethyl-2-oxovaleriat, Ethyl-2-oxo-4-phenylbutyrat, Ethylpyruvat, Ethylphenylglyoxylat, Ethyl-2-oxo-3-phenylpropiosäure, Ethyl-8-chloro-6-oxooctanoat, Ethyl-2-oxobutyrat, Ethyl-2-oxohexanoat, Methylphenylglyoxylat, Methyl-2-oxovaleriat, Methylpyruvat, Methyl-2-oxo-4-phenylbutyrat, Methyl-2-oxo-3-phenylpropiosäure, Methyl-8-chloro-6-oxo-octanoat, Methyl-2-oxobutyrat oder Methyl-2-oxo-hexanoat.

Die entsprechend gebildeten S-2-Hydroxysäureester sind beispielsweise Ethyl-S-2-Hydroxy-valeriat, Ethyl-S-2-Hydroxy-4-phenylbutyrat, Ethyl-L-Lactat oder Ethyl-S-mandelat.

Geeignete Oxidoreduktasen stammen beispielsweise aus Lactobacillus reuteri. Die Oxidoreduktase kann in dem erfindungsgemäßen Verfahren entweder vollständig gereinigt oder teilweise gereinigt eingesetzt werden oder in Zellen enthaltend verwendet werden. Die eingesetzten Zellen können dabei nativ, permeabilisiert oder lysiert vorliegen. Bevorzugt wird die klonierte Oxidoreduktase gemäß SEQ ID NO: 18 eingesetzt.

Die Volumenaktivität der eingesetzten Oxidoreduktase beträgt von 250 Units/ml (U/ml) bis 20000 U/ml, bevorzugt etwa 4000 U/ml. Je kg umzusetzender Verbindung der Formel I werden 5000 bis 250000 U Oxidoreduktase eingesetzt, bevorzugt etwa 10000 U bis 50000 U. Der Enzymeinheit 1 U entspricht dabei der Enzymmenge, die benötigt wird um 1 µmol von Ethyl-2-oxo-phenyl-butyrat zu S-Ethyl-2-hydroxy-4-phenylbutyrat je Minute (min) umzusetzen.

Die Erfindung betrifft ferner ein Verfahren zur enantioselektiven Gewinnung von S-2-Hydroxysäureester, das dadurch gekennzeichnet ist, dass man

a) 2-Oxosäureester in Anwesenheit von Oxidoreduktase, NADPH und Wasser zum entsprechenden S-2-Hydroxysäureester reduziert,
b) das durch die Oxidoreduktase gebildete NADP mit einer Dehydrogenase und einem Cosubstrat gleichzeitig zu NADPH reduziert, und
c) den gebildeten chiralen S-2-Hydroxysäureester isoliert.

Geeignete Dehydrogenasen sind beispielsweise Alkohol-Dehydrogenasen aus Thermoanaerobium brockii, Lactobacillus kefir oder Lactobacillus brevis, wobei diese Enzyme als Coenzym NADPH benötigen (DE 19 610 984, EP 0 456 107, WO 97/32012).

Geeignete Cosubstrate für die eingesetzte Alkohol-Dehydrogenase sind Alkohole wie Ethanol, 2-Propanol (Isopropanol), 2-Butanol, 2-Pentanol oder 2-Octanol.

Ferner kann die NADP-Reduktion auch mit den bekannten zur Regenerierung von NADPH verwendeten Enzymen durchgeführt werden, beispielsweise mit Glucose-Dehydrogenase oder NADPH abhängige Formiat-Dehydrogenase (Tishkov et al., J. Biotechnol. Bioeng. [1999] 64, 187-193, Pilot-scale production and isolation of recombinant NAD and NADP specific formate dehydrogenase).

Das geeignete Cosubstrat für das erfindungsgemäße Verfahren ist bei Einsatz der Glucose-Dehydrogenase Glucose. Geeignete Cosubstrate der Formiat-Dehydrogenase sind beispielsweise Salze der Ameisensäure wie Ammoniumformiat, Natriumformiat oder Calziumformiat.

Bevorzugt wird die Alkohol-Dehydrogenase aus Lactobacillus minor (DE 101 19274) eingesetzt. Die Alkohol-Dehydrogenase kann in dem erfindungsgemäßen Verfahren entweder vollständig gereinigt oder teilweise gereinigt eingesetzt werden oder es können ganze Zellen, die die Alkohol-Dehydrogenase enthalten, verwendet werden. Die eingesetzten Zellen können dabei nativ, permeabilisiert oder lysiert vorliegen. Je kg umzusetzender Verbindung der Formel I werden 10 000 U bis 200 000 U Alkohol-Dehydrogenase eingesetzt, bevorzugt etwa 25 000 U bis 100 000 U. Der Enzymeinheit 1 U entspricht dabei der Enzymmenge die benötigt wird um 1 µmol des Cosubstrates (z.B. 2-Propanol) je Minute (min) umzusetzen.

Dem Wasser wird bevorzugt ein Puffer zugesetzt, beispielsweise Kaliumphosphat-, Tris/HCl- oder Triethanolamin-Puffer mit einem pH-Wert von 5 bis 10, vorzugsweise ein pH-Wert von 6 bis 9. Die Pufferkonzentration beträgt von 10 mM bis 150 mM, bevorzugt von 90 mM bis 110 mM, insbesondere 100 mM. Der Puffer kann zusätzlich noch Ionen zur Stabilisierung oder Aktivierung beider Enzyme enthalten, beispielsweise Magnesiumionen zur Stabilisierung der Alkohol-Dehydrogenase aus Lactobaillus minor.

Die Temperatur beträgt bei den erfindungsgemäßen Verfahren beispielsweise von etwa 10 °C bis 60 °C, bevorzugt von 30 °C bis 55 °C.

Gemäß einer bevorzugten Ausführungsform des Verfahrens werden die Reaktionen in Anwesenheit eines organischen Lösungsmittels durchgeführt.

Die bevorzugten organischen Lösungsmittel sind beispielsweise Diethylether, tertiär-Butylmethylether, Diisopropylether, Dibutylether, Butylacetat, Heptan, Hexan oder Cyclohexan.

Der Reaktionsansatz besteht beim Einsatz zusätzlicher Lösungsmittel aus einer wässrigen Phase und einer organischen Phase. Die organische Phase wird durch ein geeignetes Lösungsmittel in dem das Substrat gelöst vorliegt oder wird durch das wasserunlösliche Substrat selbst gebildet. Die organische Phase beträgt dabei von etwa 5 % bis 80 % des gesamten Reaktionsvolumens, bevorzugt von 10 % bis 40%.

Das Wasser bildet im erfindungsgemäßen Zwei-Phasen-System aus einer ersten flüssigen Phase und dem organischen Lösungsmittel die zweite flüssige Phase. Gegebenenfalls kann auch noch eine feste oder weitere flüssige Phase vorliegen, die beispielsweise durch nicht vollständig gelöste Oxidoreduktase und/oder Alkohol-Dehydrogenase oder durch die Verbindung der Formel I entsteht. Bevorzugt sind jedoch zwei flüssige Phasen ohne feste Phase. Die zwei flüssigen Phasen werden bevorzugt mechanisch gemischt, damit große Oberflächen zwischen den beiden flüssigen Phasen erzeugt werden.

Die Konzentration des Cofaktors NADPH bezogen auf die wässrige Phase beträgt von 0,001 mM bis 0,1 mM, insbesondere von 0,005 mM bis 0,02 mM.

Bevorzugt wird im erfindungsgemäßen Verfahren noch ein weiterer Stabilisator der Alkohol-Dehydrogenase eingesetzt. Geeignete Stabilisatoren sind beispielsweise Glycerin, Sorbitol oder Dimethylsulfoxid (DMSO).

Die Verbindungen der Formel I werden im erfindungsgemäßen Verfahren in einer Menge von 10 % bis 60 % bezogen auf das Gesamtvolumen eingesetzt, bevorzugt von 15 % bis 50 %, insbesondere von 20 % bis 40 %.

Die Menge des Cosubstrats für die Regenerierung von NADP zu NADPH wie Isopropanol beträgt von etwa 5 % bis 50 % bezogen auf das Gesamtvolumen, bevorzugt von 10 % bis 30 %, insbesondere von 15 % bis 25 %.

Das erfindungsgemäße Verfahren wird beispielsweise in einem geschlossen Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Je nach Substrat und eingesetzter Verbindung der Formel I beträgt die Reaktionszeit von 1 Stunde bis 48 Stunden, insbesondere von 2 Stunden bis 24 Stunden.

Anschließend wird das Reaktionsgemisch aufgearbeitet. Dazu wird die wässrige Phase abgetrennt, die organische Phase wird gefiltert. Die wässrige Phase kann gegebenenfalls noch einmal extrahiert werden und wie die organische Phase weiter aufgearbeitet werden. Danach wird gegebenenfalls das Lösungsmitel aus der klaren organischen Phase verdampft. Man erhält so beispielsweise das Produkt S-Ethyl-2-hydroxy-4-phenylbutyrat mit einer Enantiomerenreinheit von mehr als 94 % und das im wesentlichen frei vom Edukt Ethyl-2-oxo-4-phenylbutyrat ist. Die Gesamtausbeute der Prozesses beträgt nach Destillation des Produktes von 50 % bis 95 % bezogen auf die eingesetzte Eduktmenge.

Die Erfindung wird durch die folgenden Beispiele erläutert:

Beispiel 1 Screening nach Oxidoreduktasen zur Reduktion von 2-Oxosäureestern in Stämmen der Gattung Lactobacillus

Zum Screening wurden verschiedene Stämme der Gattung Lactobacillus in folgendem Medium kultiviert (Angaben jeweils g/l): Glucose (20), Hefeextrakt (5), Fleischextrakt (10), Di-Ammoniumhydrogencitrat (2), Natriumacetat (5), Magnesiumsulfat (0,2), Mangansulfat (0,05), Di-Kaliumhydrogenphosphat (2). Das Medium wurde bei 121 °C sterilisiert und die Stämme der Gattung Lactobacillus (im folgenden mit L. abgekürzt) wurden ohne weitere pH-Regulierung oder Sauerstoffzufuhr kultiviert.

Anschließend wurden 125 mg Zellen mit 800 µl Aufschlusspuffer (100 mM Triethanolamin (TEA), pH 7,0) resuspendiert, mit 1 g Glasperlen versetzt und 10 min bei 4 °C in der Kugelmühle aufgeschlossen (Retsch). Der nach 2 Minuten (min) Zentrifugation bei 12000 Umdrehungen pro Minute (rpm) erhaltene Überstand wurde im AKtivitätsscreening und zur Bestimmung des Enatiomerenüberschuss eingesetzt. Als Substrate wurden Ethyl-2-oxopentanoat und Ethyl-2-oxo-4-phenylbutyrat eingesetzt.

<u>Ansatz Aktivitätsscreening:</u>

| | |
|---|---|
| 860 µl | 0,1 M $KH_2PO_4/K_2PO_4$ pH = 7,0 1mM $MgCl_2$ |
| 20 µl | NADPH/NADH (10 mM) |
| 20 µl | Lysat |
| 100 µl | Substrat (100 mM) |

Die Reaktion wurde 1 min bei 340 nm verfolgt.

<u>Ansatz ee-Wert Bestimmung</u>

| | |
|---|---|
| 20 µl | Lysat |
| 100 µl | NADH/NADPH (50 mM) |
| 60 µl | Substrat ( Ethyl-2-oxo-4-phenylbutyrat 100 mM) |

Die Ansätze zur ee- Bestimmung wurden nach 24 Stunden (h) mit Chloroform extrahiert und mittels GC der Enantiomerenüberschuss analysiert.

Der Enantiomerenüberschuß berechnet sich wie folgt:

$$ee(\%) = ((R\text{-Alkohol} - S\text{-Alkohol})/(R\text{-Alkohol} + S\text{-Alkohol})) \times 100.$$

Tabelle 1.

| DSMZ Nr. | Name | Ethyl-2-oxo-4-phenylbutyrat Aktivität in U/g Zellen Wirtsorganismus | | | Ethyl-2-oxopentanoat Aktivität in U/g Zellen Wirtsorganismus | |
|---|---|---|---|---|---|---|
| | | NADH | NADPH | ee-Wert | NADH | NADPH |
| 20011 | L. casei | 0 | 0 | --- | 0 | 0 |
| 20019 | L. curvatus var. Curvatus | 0 | 0 | --- | 0 | 0 |
| 20184 | L. farciminis | 0 | 0 | --- | 0 | 0 |
| 20243 | L. gasseri | 0 | 0 | --- | 0 | 0 |
| 20249 | L. alimentarius | 0 | 0 | --- | 0 | 0 |
| 20494 | L. sakei | 0 | 0 | --- | 0 | 0 |
| 20555 | L. salivarius var. Salivarius | 0 | 0 | --- | 0 | 0 |
| 20557 | L. jensenii | 0 | 0 | --- | 0 | |
| 20074 | L. delbrueckii var. Delbrueckii | 0 | 0 | --- | 0 | 0 |
| 20001 | L. coryniformis var. caryniformis | 0 | 0 | --- | 0 | 0 |
| 20190 | L. halotolerans | 0 | 0 | --- | 0 | 0 |
| 20016 | L. reuteri | 0 | 12,3 | 94 % S | 0 | 21,1 |
| 20003 | L. bifermentans | 0 | 0 | --- | 0 | 0 |
| | L. kefiri | 0 | 2,5 | 26 % S | 0 | 7,7 |
| 4864 | L. oris | 0 | 0 | --- | 0 | 0 |
| 20515 | L. collinoides | 2,6 | 3,4 | 26 % R | 2,5 | 7,7 |
| 20014 | L. minor | 0 | 0 | --- | 0 | 0 |
| 20593 | L. kandleri | 3,6 | 3,6 | 32 % S | 4,3 | 12,8 |
| 5705 | L. parabuchneri | 0 | 5,1 | 38 % S | 0 | 11,3 |
| 20349 | L. fructosus | 0 | 0 | --- | 0 | 0 |
| 20055 | L.cellobiosus | 0 | 12,3 | 92 % S | 0 | 13,3 |
| 20015 | L.reuteri | 0 | 12 | 96,6 % S | 0 | 26 |
| 20049 | L.fermentum | 0 | 7,7 | 82 % S | 0 | 6,8 |
| 20052 | L.fermentum | 0 | 0 | --- | 0 | 0 |

DSMZ steht für Deutschen Sammlung für Mikroorganismen und Zellkulturen, Mascheroder Weg 1b, 38124 Braunschweig.

Aus der Tabelle 1 ergibt sich, dass in der Gattung Lactobacillus mehrere Arten eine NADPH abhängige Oxidoreduktase mit Ethyl-2-oxo-4-phenylbutyrat oder Ethyl-2-oxopentanoat als Substrat aufweisen.

Definition der Enzymeinheiten: 1 U entspricht der Enzymmenge die benötigt wird um 1 μmol Substrat pro min

umzusetzen.

Beispiel 2: Isolierung einer NADPH abhängigen Oxidoreduktase aus Lactobacillus reuteri

Zur Isolierung einer NADPH abhängigen Oxidoreduktase aus Lactobacillus reuteri wurde der Organismus wie unter Beispiel 1 beschrieben kultiviert. Nach Erreichen der stationären Phase wurden die Zellen geerntet und mittels Zentrifugation vom Medium getrennt. Die Enzymfreisetzung erfolgte durch Naßvermahlung mittels Glasperlen, hätte aber auch durch andere Aufschlussmethoden erreicht werden. Dazu wurden 20g L. reuteri mit 80 ml Aufschlusspuffer (100 mM Triethanolamin, 1 mM $MgCl_2$ pH =7,0) suspendiert und nach Zugabe von 80 ml Glasperlen erfolgte der Zellaufschluss mittels Kugelmühle (Retsch, 10 min , 24 Hz).

Der nach Zentrifugation erhaltene Rohextrakt wurde dann durch Zugabe von 242 mg $(NH_4)_2SO_4$ auf eine Endkonzentration von 50 % Ammoniumsulfat eingestellt und 1 h bei 4 °C gerührt. Anschließend wurde das Pelett bei 12000 rpm für 10 min abzentrifugiert und der erhaltene Überstand mittels FPLC weiter aufgereinigt. Das Enzym wurde mit hydrophober Interaktionschromatographie an Butylsepharose Fast Flow (Pharmacia) und anschließender Gelpermeation (Superdex 200 HR, Pharmacia) aufgereinigt. Dazu wurde der Überstand nach Ammoniumsulfatfällung direkt auf eine mit 100 mM TEA pH = 7,0 und 1 M $(NH_4)_2SO_4$ äquilibrierte Butylsepharose FF-Säule aufgetragen und mit einen fallenden linearen Salzgradienten eluiert. Das Enzym wurde dabei bei 0 M $(NH_4)_2SO_4$ eluiert. Die aktiven Fraktionen wurden vereinigt und mittels Ultrafiltration (Ausschlußgrenze 10 kDa) auf ein geeignetes Volumen eingeengt. Die Enzymaktivität der Oxidoreduktase wird im Testsystem gemäß Beispiel 1, (Ansatz Aktivitätsscreening) bestimmt und die Bestimmung der Proteinmenge erfolgte gemäß Lowry et al. Journal of Biological Chemistry, 193 (1951): 265-275 oder Peterson et al. , Analytical Biochemistry, 100 (1979): 201-220). Der Quotient von Enzymaktivität zu Proteinmenge ergibt die spezifische Aktivität, wobei der Umsatz von 1 $\mu$mol pro min 1 Unit (U) entspricht.

Anschließend wurde die Rohenzympräperation mittels Gelpermeation ( TEA 100 mM pH = 7,0, 0,15 M NaCl, 1mM $MgCl_2$) weiter aufgereinigt und gleichzeitig das Molekulargewicht des nativen Enzyms bestimmt. Als Molekulargewichtsstandards wurden Catalase (232 kDa), Aldolase (158 kDa), Albumin (69,8kDa) und Ovalbumin (49,4 kDa) verwendet.

Reinigungstabelle

Tabelle 2

| Reinigungsschritt | Volumen [ml] | Aktivität [U/ml] | Gesamtaktivität [U] | Spezifische Aktivität [U/mg] | Ausbeute |
|---|---|---|---|---|---|
| Rohextrakt | 20 | 9,3 | 186 | 0,25 | 100 % |
| $(NH_4)_2SO_4$ - Fällung | 20 | 5,4 | 109 | 0,83 | 57 % |
| Butylsepharose | 1 | 20 | 20 | 54 | 10 % |
| Gelpermeation | 0,1 | 20 | 2 | 120 | 1,1 % |

Das mittels Gelpermation bestimmte Molekulargewicht des Proteins im nativen Zustand beträgt 60 $\pm$5 kDa.

Beispiel 3: Bestimmung der N-terminalen Sequenz und Bestimmung eines internen Peptides nach In-Gelverdau

Die Enzympräparation wurde nach der Gelpermeation im 10 % igen Natriumdodecylsulfat (SDS) Gel aufgetrennt und auf eine Polyvinyliden Diflourid-Membran (PVDF-Membran) übertragen.

Die auffällige Bande bei etwa 30 bis 35 kDa wurde einer N-terminalen Sequenzierung mittels Edman-Abbau (Procise 492 (PE-Biosystems) unterworfen. Es wurde folgende N-terminale Sequenz erhalten:

SEQ ID 1:
M K N I M I A G A G V L G S Q

Die SDS-PAGE- Bande des selben Proteins wurde mit Dithiothreitol reduziert, carboxymethyliert und mit Endoproteinase Lys-C verdaut. Die erhaltenen Peptide wurden über eine 300 $\mu$m x 150 mm Kapillar-HPLC-Säule (Vydac RP18, LC Packings getrennt. 25 Fraktionen wurden manuell gesammelt und mittels MALDI MS (Voyager-DE STR (PE-Biosystems) auf für die Sequenzierung geeignete Peptide geprüft. Die Fraktion 12 mit MH+ = 1491.8 wurde mittels automatischem Edman-Abbau sequenziert und lieferte folgende Sequenz:

SEQ ID 2:
S D Y E R D L H L T D K

Beispiel 4 : Klonierung des Enzyms

4.1 Klonierung eines spezifischen Genfragments von L.reuteri mit Hilfe von PCR ( Polymerase chain reaction).

Chromosomale DNA wurde aus den Zellen von Lactobacillus reuteri nach der in "Molecular cloning" von Maniatis & Sambrook beschriebenen Methode extrahiert. Die resultierende genomische DNA diente als Matrize für die direkte Polymerasen Kettenreaktion (PCR) mit degenerierten Primern. Dabei wurden die degenerierten 5'-Primer von der N-terminalen Aminosäurensequenz (Seq. No: 1) und die 3'-Primer von der Aminosäuresequenz eines intern liegenden Peptides (Seq. No: 2) unter Einbeziehung des universellern Genkodes abgeleitet (Seq No:3, 4, 5 und 6).
Primerkonstrukte sind im folgenden aufgelistet
N = A,T, C oder G; Y = T oder C; R = A oder G
Die Primer wurden nach bekannten Verfahren hergestellt.
5'-Oligo 3:
ATGAARAAYATYATGATYGCHGGCGC
5'-Oligo 4:
ATGAARAAYATYATGATYGCHGGTGC
3'-Oligo 5:
RTGHARATCMCGTTCRTAATC
3'-Oligo 6:
RTGHARATCMCGTTCRTAGTC

Die Amplifizierung wurde in PCR-Puffer [10 mM Tris-HCl (pH 8,3), 50 mM KCl, 2,5 mM $MgCl_2$, 1 mM Desoxy-Nukleotidtriphosphat Mix (dNTP) Mix, 30 pMol je Primer und 2,5 U AmpliTaq Gold (Applied Biosystems) durchgeführt. Nach einer Aktivierung der AmpliTaq Gold Polymerase (10 min, 94 °C) und folgenden 35 Zyklen PCR (94 °C, 60 sec ; 53 °C, 45 sec; 72 ° C, 60 sec) wurde die Reaktion auf 4° C abgekühlt und der gesamte PCR-Ansatz auf ein 1 % Agarose Gel zur Analyse aufgetragen.

4.2 Subklonierung von PCR Amplifizierungsprodukt

Ein bei 200 bp identifiziertes spezifisches Fragment des L. reuteri (S)-ADH Genes wurde nach der Gelreinigung (Qiaex Agarose extraktion kit) in den TA-Cloning Vector pCR 2.1 (Invitrogen, Karlsruhe, Deutschland) ligiert. Nach der Transformation von 2 µl der Ligationsansatzes in E.coli Top 10F' Zellen wurden DNA's entstandener Kolonien auf das Vorhandensein des Plasmids pCR2.1 mit integriertem 200 bp PCR-Fragment gescreent. Dazu wurde eine Restriktionsanalyse mit Eco RI Endonuklease durchgeführt. Anschließend wurden positive Klone mit den folgenden Primern:

M13 rev: 5' CAGGAAACAGCTATGACC 3' und
M13 uni: 5' TGTAAAACGACGGCCAGT 3'
mit Hilfe von ABI DNA Sequenzer sequenziert.

Sequenzanalyse des 159 bp langen Genfragmentes (Seq. No: 7) zeigte einen offenen Leserrahmen von 53 Aminosäuren, in den auch beide Sequenz-Fragmente des N-Terminus und des internes Peptides wieder zu finden waren.

4.3 Klonierung von für L.reuteri (S)-ADH kodierendem volllänge-Gensegment Basierend auf der Nukleotidsequenz des 159 bp langen Genfragment aus dem Beispiel 4.2. wurden spezifische Primerpaare für eine inverse Polymerase Kettenreaktion (iPCR) mit nachfolgender Nest-PCR konstruiert (Seq. No: 8, 9, 10 und 11). Dabei sind die Primer 8 und 9 komplementär zum 3' Ende des gefundenen Genfragments, und die Primer 10 und 11 sind zu der Region in der Nähe des 5' Ende komplementär.
Oligo 8:
ATCCGGCTTTAATGTCAGCGT
Oligo 9:
CGACGGATTAAGGCGCTGAAAAG
Oligo 10:
CTACCTAATACGCCAGCACCAG
Oligo 11:
GCCAGCACCAGCAATCAT

Chromosomale DNA aus Zellen von L. reuteri wurde mit Eco RI Endonuklease verdaut und zur Religation mit T4 Ligase eingesetzt. Die resultierenden zirkulären, chromosomalen DNA-Fragmente dienten als Matrize für die iPCR. Folgende Amplifizierungszyklen einer Polymerasen Kettenreaktion wurden in einem PCR-Buffer (s. Beispiel 2), 1 mM $MgCl_2$, mit je 30 pMol von Primern 8 und 10, 25 ng des Religationsproduktes als Template und 2,5 U AmpliTaq Gold DNA Polymerase (Applied Biosystems) durchgeführt:

| | |
|---|---|
| Zyklus 1: | 94 ° C, 10 min |
| Zyklus 2 x 30: | 94 °C, 1 min |
| | 57 ° C, 45 sec |
| | 72 ° C, 1 min |
| Zyklus 3: | 72 ° C, 7 min |
| | 4 ° C, ∞ |

Das PCR-Signal wurde anschließend durch eine Nest-PCR verstärkt. Die optimale $MgCl_2$ Konzentration lag in dieser Reaktion bei 2 mM und 2 µl der ersten iPCR als Template. Durch eine Gradienten-PCR wurde das Temperatur-Optimum für das Primerpaar 9 und 11 bei 57 °C bestimmt. Folgende Amplifizierungszyklen waren mit AmpliTaq Gold DNA Polymerase notwendig, um ein spezifisches PCR-Produkt von 1200 bp Länge detektieren zu können:

| | |
|---|---|
| Zyklus 1: | 95 ° C, 10 min |
| Zyklus 2 x 40: | 95 ° C, 45 sec |
| | 57 ° C, 1 min |
| | 72 ° C, 90 sec |
| Zyklus 3: | 72 ° C, 7 min |
| | 4 ° C, ∞ |

Die spezifische Bande mit einer Länge von 1200 bp wurde über ein 1 % Agarose Gel mittels Qiaex Gel Extraktionskit (Qiagen, Hilden, Deutschland) gereinigt und in einer Ligationsreaktion mit dem TA-PCR-Kloning Vektor pCR2.1 (Invitrogen) eingesetzt.

Nach der Transformation von 2 µl des Ligationsansatzes in E.coli Top 10F' Zellen wurden Plasmid DNA's entstandener Ampicillin-resistenter Kolonien auf das Vorhandensein des Plasmids pCR2.1 mit integriertem 1200 bp PCR-Fragment gescreent. Dazu wurde eine Restriktionsanalyse mit Eco RI Endonuklease durchgeführt. Anschließend wurden positive Klone mit den Primern M13 rev und M13 uni wie unter 4.2 beschrieben sequenziert.

Die Sequenzanalyse des 1241 bp langen DNA-Fragments (Seq. No: 12) zeigte im 5'-terminalen Bereich einen offenen Leserrahmen von 148 Aminosäuren. Die Sequenz der ersten 15 N-terminalen Aminosäuren stimmte mit der C-terminalen Aminosäurensequenz Seq No: 7 aus 4.2 überein. Die Analyse der C-terminalen Sequenz des 1241 bp langen DNA-Fragmentes zeigte einliegende regulatorische DNA-Segmente an das N-terminale Ende der (S)-ADH Gens von L.reuteri

Basierend auf der Sequenz des 1241 bp langen DNA-Fragment, dessen N-terminaler Bereich (447 bp) einen weiteren Genabschnitt der Oxidoreduktase aus L. reuteri darstellt, wurden spezifische Primer für eine weitere inverse Polymerasen Kettenreaktion (iPCR) mit nachfolgender Nest-PCR konstruiert (Seq. No: 13 und 14). Primer 13 und 14 sind komplementär zur 3' Verlängerung des gefundenen Genfragments.
Oligo 13:
CCAGAGGTGATTGAAGAAGCTAC
Oligo 14:
CCGGGAAATAAAGATGGTT

Chromosomale DNA aus Zellen von L. reuteri wurde mit Afl III Endonuklease verdaut und zur Religation mit T4 Ligase eingesetzt. Die resultierenden zirkulären chromosomalen DNA-Fragmente dienten als Matrize für die iPCR. Folgende Amplifizierungszyklen einer Polymerasen Kettenreaktion wurden in einem PCR-Buffer (s. Beispiel 4.1),1mM $MgCl_2$, mit je 30 pMol von Primern 7a/9a, 25 ng des Religationsproduktes als Template und 2,5 U AmpliTaq Gold DNA Polymerase (Applied Biosystems) durchgeführt:

| | |
|---|---|
| Zyklus 1: | 94 ° C, 10 min |
| Zyklus 2 x 30: | 95 °C, 1 min |
| | 56 ° C, 45 sec |
| | 72 ° C, 1:45 min |

(fortgesetzt)

| | |
|---|---|
| Zyklus 3: | 72 ° C, 7 min |
| | 4 ° C, ∞ |

Das PCR-Signal wurde anschließend durch eine Nest-PCR verstärkt. Die optimale $MgCl_2$ Konzentration lag in dieser Reaktion bei 2 mM und 2 µl der ersten iPCR wurden als Template für die Nest-PCR benutzt. Folgende Amplifizierungszyklen waren mit AmpliTaq Gold DNA Polymerase notwendig um ein spezifisches PCR-Produkt von 950 bp Länge detektieren zu können:

| | |
|---|---|
| Zyklus 1: | 94 ° C, 10 min |
| Zyklus 2 x 40: | 94 ° C, 45 sec |
| | 56 ° C, 1 min |
| | 72 ° C, 1:45 min |
| Zyklus 3: | 72 ° C, 7 min |
| | 4 ° C, ∞ |

Eine spezifische Bande mit einer Länge von 950 bp wurde über ein 1 % Agarose Gel mittels Qiaex Gel Extraktionskit (Qiagene) gereinigt und in einer Ligationsreaktion mit dem TA-PCR-Kloning Vektor pCR2.1 (Invitrogen) eingesetzt.

Nach der Transformation von 2 µl des Ligationsansatzes in E.coli Top 10F' Zellen wurden Plasmid DNA's entstandener Ampicillin-resistenter Kolonien auf das Vorhandensein des Plasmids pCR2.1 mit integriertem 950 bp PCR-Fragment gescreent. Dazu wurde eine Restriktionsanalyse mit Eco RI Endonuklease durchgeführt. Anschließend wurden positive Klone mit den Primern M13 rev und M13 uni wie im 4.2 beschrieben sequenziert.

Das in den pCR2.1 Vektor inserierte DNA-Fragment war 822 bp lang und besaß im N-terminus einen offenen Leserrahmen von 126 Aminosäuren, der mit einem Stopcodon und einer Terminationsschleife endete (Seq No: 15). Das 5'-Peptid von 12 Aminosäuren stimmte mit dem C-Terminalen Ende der Sequenz No: 12 überein. Somit enthielt das durch iPCR generierte DNA-Fragment von 822 bp das C-Terminale Ende des für eine Oxidoreduktase von L. reuteri kodierenden Gensegmentes.

4.4 Synthese des vollen Gens einer Oxodoreduktse aus L. reuteri mittels PCR

Basierend auf den Sequenzen No: 7 und No: 15 wurden spezifische Primer für eine nachfolgende Klonierung des Vollängen-Gen in ein passendes Expressionssystem konstruiert. Dabei wurde der 5'-Primer mit einer Erkennungssequenz für Nde I und 3'-Primer mit einer Erkennungssequenz für Hind III modifiziert (Seq. No: 16; Seq. No: 17)
Oligo 16:
GCGGAATTCCATATGAAGAATATCATGATTGCT
Oligo 17:
CCCAAGCTTAATGCTTCAGAAAATCTGG

Genomische DNA aus den Zellen von L. reuteri diente als Matritze für die Polymerasen Ketten-Reaktion. Amplifiziert wurde in einem PCR-Puffer [10 mM Tris-HCl, (pH 8,0); 50 mM KCl; 10 mM $MgSO_4$; 1 mM dNTP Mix; je 30 pMol Primer und 2,5 U Platinum Pfx DNA-Polymerase (Invitrogen)] mit 300 ng Template und mit folgenden Temperatur-Zyklen :

| | |
|---|---|
| Zyklus 1: | 94 ° C, 2 min |
| Zyklus 2 x 30: | 94 ° C, 15 sec |
| | 58 ° C, 30 sec |
| | 68 ° C, 75 sec |
| Zyklus 3: | 68 ° C, 7 min |
| | 4 ° C, ∞ |

Das resultierende PCR-Produkt wurde nach der Reinigung über ein 1 % Agarose Gel mit Nde I und Hind III verdaut und in das mit den gleichen Endonukleasen behandelten Rückgrad des pET21a Vectors (Novogene, Madison, USA) ligiert. Nach der Transformation von 2 µl des Ligationsansatzes in E.coli Top 10 F' Zellen wurden Plasmid-DNA's Ampicillin-resistenter Kolonien mittels einer Restriktionsanalyse mit Endonukleasen Nde I und Hind III auf die Richtigkeit der erfolgten Ligation überprüft. Das Expressionskonstrukt pET21-reut#10 wurde sequenziert. Das Oxidoreduktasegen von Lactobacillus reuteri besitzt einen offenen Leserrahmen von insgesamt 882 bp (Seq. No: 19) was einem Protein von 294 Aminosäuren entspricht (Seq. No: 18)

4.5 Produktion von rekombinanter (S)-ADH in E.coli

Kompetente Escherichia coli StarBL21 (De3) Zellen (Invitrogen) wurden mit dem das Oxidoreduktase-Gen enthaltendem Expressionskonstrukt pET21-reut#10 transformiert. Der Stamm wurde in LB Medium (1 % Tryptone, 0.5 % Hefeextrakt, 1 % NaCl) mit Ampicillin (50 μg/ml) kultiviert, bis eine optische Dichte gemessen bei 500 nm von 0,5 erreicht wurde. Die Expression der Oxidoreduktase wurde durch Zugabe von Isopropylthiogalaktosid (IPTG) in einer Endkonzentration von 1 mM induziert. Nach 8 Stunden Induktion bei 25 ° C und 220 rpm wurden die Zellen geerntet und bei -20 °C eingefroren.

Für die folgenden Versuche zur biochemischen Charakterisierung wurden 100 mg Zellen mit 600 μl Aufschlusspuffer und 600 μl Glasperlen versetzt und 10 min mittels Kugelmühle aufgeschlossen. Das erhaltene Lysat wurde dann verdünnt für die entsprechenden Messungen eingesetzt. Dabei hatte das Lysat eine Aktivität mit Ethyl-2-oxo-4-phenylbutyrate von 2000 bis 4000 U/ml.

Das Enzym konnte also mit Aktivitäten von 10000 U/g bis 30000 U/g Feuchtgewicht E.coli exprimiert werden. Damit ist das Enzym preiswert und in großen Mengen verfügbar.

Beispiel 5: Charakterisierung der rekombinanten Oxidoreduktase aus L. reuteri 5.1 pH- Optimum Herstellung folgender Messpuffer alle 50 mM und mit 1 mM $MgCl_2$ mit unterschiedlichen pH - Werten

Tabelle 3

| pH-Wert | Puffersystem | pH-Wert | Puffersystem |
|---|---|---|---|
| 4 | Na-acetat/Essigsäure | 7,5 | $KH_2PO_4/K_2PO_4$ |
| 4,5 | Na-acetat/Essigsäure | 8 | $KH_2PO_4/K_2PO_4$ |
| 5 | Na-acetat/Essigsäure | 8,5 | $KH_2PO_4/K_2PO_4$ |
| 5,5 | $KH_2PO_4/K_2PO_4$ | 9 | Glycin/NaOH |
| 6 | $KH_2PO_4/K_2PO_4$ | 9,5 | Glycin/NaOH |
| 6,5 | $KH_2PO_4/K_2PO_4$ | 10 | Glycin/NaOH |
| 7 | $KH_2PO_4/K_2PO_4$ | 11 | Glycin/NaOH |

Verdünnung des Enzyms nach Bedarf (1:20)

Meßansatz (30 °C):

| | |
|---|---|
| 870 μl | Meßpuffer mit variierenden pH |
| 20 μl | NADPH 10 mM (8,6 mg/ml $H_2O$ ) |
| 10 μl | Enzym verdünnt |
| 2-3 min Inkubation | |
| + 100 μl | Substratlösung (100 mM Ethyl-2-oxo-4-phenylbutyrat) |

Zur Bestimmung des pH-Optimums wurde die enzymatische Reaktion in dem jeweiligen in Tabelle 3 aufgeführten Puffer bestimmt. Dabei konnte für das erfindungsgemäße Enzym ein pH-Optimum zwischen 6,5 und 7 ermittelt werden. Im pH -Bereich von 4,5 bis 8 hat das Enzym 80% seiner maximalen Aktivität, bei pH-Werten von unter 4,0 und über 8,5 sinkt die Aktivität dann rapide ab.

5.2 pH-Stabilität

Die Abhängigkeit der Aktivität des Enzyms bei Lagerung in Puffern mit verschiedenen pH-Werten wurde im Bereich von pH 4 bis 11 untersucht. Dazu wurden verschiedene Puffer (50mM) im Bereich von pH 4 bis 11 angesetzt und das in Beispiel 4 überexprimierte Enzym darin 1:200 verdünnt und 30, 60 und 300 min inkubiert. Alle Puffer enthielten 1mM $MgCl_2$. Anschließend wurden davon 10 µl im normalen Aktivitätstest eingesetzt. Ausgangswert ist dabei der Messwert, den man unmittelbar nach Verdünnung des Enzyms in Kaliumphosphatpuffer 50 mM pH = 7.0 erhält. Dieser Wert entsprach unter vorgegeben Bedingungen einer Extinktionsänderung von 0,70 /min und wurde als 100 %-Wert gesetzt und alle folgenden Messwerte wurden zu diesem Wert ins Verhältnis gesetzt.

Dabei wurde festgestellt, dass die rekombinante Oxidoreduktase aus L. reuteri im pH- Bereich von 4,5 bis 8,0 stabil ist und für mindestens 5 h ohne Aktivitätsverlust inkubiert werden kann. Bei den pH-Werten 4,0 und 9,0 wurden nach 5 h 50 % bzw. 40 % Restaktivität festgestellt. PH-Werte über 9,5 führen zu einer sofortigen Desaktivierung des Enzyms.

5.3 Temperatur-Optimum

Zur Bestimmung der optimalen Testtemperatur wurde die Enzymaktivität im Temperaturbereich von 15 °C bis 70 °C im Standardmeßansatz gemessen. Wie aus Tabelle 5 ersichtlich hat das Enzym seine maximale Aktivität bei 55°C, anschließend sinkt die Aktivität rapide ab.

Tabelle 4

| Temperatur (°C) | Aktivität in U/ml unverdünntes Enzym | Temperatur (°C) | Aktivität in U/ml unverdünntes Enzym |
|---|---|---|---|
| 15 | 385 | 45 | 2900 |
| 20 | 745 | 50 | 3200 |
| 25 | 1090 | 55 | 3800 |
| 30 | 1350 | 60 | 617 |
| 35 | 1860 | 65 | 180 |
| 40 | 2500 | 70 | 90 |

5.4 Temperatur-Stabilität

In analoger Weise wie unter 5.2 beschrieben wurde die Temperaturstabilität für den Bereich von 15 °C bis 70 °C bestimmt. Dazu wurde jeweils eine 1:200 Verdünnung des gereinigten Enzyms für 60 min und 180 min bei der jeweiligen Temperatur inkubiert und anschließend bei 30 °C mit dem obigen Testansatz gemessen. Auch hier wurde als Ausgangswert der Meßwert herangezogen, den man unmittelbar nach Verdünnung des Enzyms in Kaliumphosphatpuffer 50 mM pH = 7.0 erhält. Dieser Wert wurde auch hier als 100 %-Wert gesetzt.

Das Enzym ist dabei in einem Temperaturbereich von 15 °C bis 50 °C vollkommen stabil und zeigt nach 3 h Inkubation keinerlei Aktivitätsverlust. Bei 55 °C ist bereits nach 30 min keine Enzymaktivität mehr nachweisbar.

5.5 Substratspektrum/Enantiomerenüberschuss

Das Substratspektrum der erfindungsgemäßen Oxidoreduktase wurde bestimmt durch Messung der Enzymaktivität mit einer Reihe von Ketonen, Oxosäuren sowie deren Estern. Dazu wurde der Standard Messansatz (Beispiel 5.1) mit unterschiedlichen Substraten verwendet. Die Aktivität mit Ethyl-2-oxo-4-phenylbuyrat wurde 100% gesetzt und alle anderen Substrate wurden hierzu ins Verhältnis gesetzt. Das Enzym zeigte keine NADP abhängige Dehydrogenaseaktivität gegenüber (R) oder (S) Ethyl-2-hydroxy-4-phenylbutyrate, (R) oder (S) 4-Chloro-3-hydroxyutyrate bzw (D) oder (L) Ethyllactat.

Für die ee-Wert-Bestimmung wurden für ausgewählte Substrate folgender Reaktionsansatz durchgeführt.

| | | |
|---|---|---|
| 100 µl | NADPH | (50 mM) |
| 60 µl | Substrat | (100 mM) |

und 1 bis 2 Units Oxidoreduktase

Die Ansätze zur ee- Bestimmung wurden nach 24 h mit Chloroform extrahiert und mittels GC der Enantiomerenüberschuss des resultierenden Alkohols analysiert.

Tabelle 5.

| Substrat | Relative Aktivität % | Stereoselektivität | Substrat | Relative Aktivität % | Stereoselektivität |
|---|---|---|---|---|---|
| Ketone | | | 3-Oxo-säureester | | |
| 1-Phenyl-2-propanon | 3 | nb | Ethyl-4-chloracotoacetat | 16 | 56% R |
| 2-chloro-1-(3-chlorpheny)ethan-1-on | 0 | nb | Methyl acetoacetat | 0,3 | 78 % S |
| Acetophenon | 0 | nb | Ethyl-8-chloro-6-oxooctansäure | 190 | 94%R |
| Caprylophenon | 0 | nb | Dimethyl-3-Oxo-1,8-octandioicsäure | 42 | Racemat 50% |
| 2-Octanon | 1 | Racemat 50% | Ethyl-3-oxovaleriat | 1,3 | nb |
| 3-Octanon | 4 | nb | | | |
| Aceton | 0 | nb | | | |
| 2-Oxosäureester | | | 2-Oxosäuren | | |
| Ethyl-2-oxovaleriat | 190 | nb | 2-Oxovaleriansäure | 0 | nb |
| Ethyl-2-oxo-4-phenylbutyrat | 100 | 98 % S | 2-Oxo-3-phenyl propionsäure | 0,5 | nb |
| Ethylpyruvat | 2 | 99 % S | 2-Oxobuttersäure | 0 | nb |
| Ethylphenylglyoxylat | 2 | | | | |
| Oxidation | | | | | |
| R-2-Hydroxy-4-phenylbutyrat | 0 | nb | 2-Propanol | 0 | nb |
| S-2-Hydroxy-4-phenylbutyrat | 0 | nb | Ethyl-D-Lactat | 0 | nb |
| S-4-Chloro-3-hydroxybutyrat | 0 | nb | Ethyl-L-Lactat | 0 | nb |
| R-4-Chloro-3-hydroxybutyrat | 0 | nb | | | |

Wie aus Tabelle 5 ersichtlich werden von der rekombinanten Oxidoreduktase aus Lactobacillus reuteri insbesondere 2-Oxosäureester stereoselektiv zu den korrespondierenden 2-Hydroxysäureestern reduziert. Die korresponierenden 2-Oxosäuren wurden nicht als Substrate akzeptiert, ebenso wurden Methylketone so gut wie nicht reduziert. 3-Oxosäureester werden zum Teil reduziert aber meist nicht stereoselektiv.

5.6 Lösungsmittelstabilität

Zur Untersuchung der Enzymstabilität bei Kontakt mit organischen Lösungsmitteln wurde die Oxioreduktase aus L. reuteri mit den angegebenen Lösungsmittelgemischen 1: 400 (bei wassermischbaren organischen Lösungsmitteln) verdünnt und bei Raumtemperatur inkubiert. Anschließend wurden 10 µl der Enzymlösung im Standardtestansatz eingesetzt. Auch hier wurde der Ausgangswert nach Verdünnung im Puffer (Kaliumphosphatpuffer 100 mM, pH = 7.0, 1 mM $MgCl_2$) gleich 100 % gesetzt und alle weiteren Werte zu diesem ins Verhältnis gesetzt..
Bei den nicht mit Wasser mischbaren organischen Lösungsmittel erfolgte die Verdünnung ebenfalls in Kaliumphosphatpuffer, es wurde das gleiche Volumen organisches Lösungsmittel zum Ansatz gegeben und der Ansatz bei Raumtemperatur im Thermomixer bei rpm = 170 inkubiert. Die Aktivitätsmessung erfolgte aus der wäßrigen Phase.

Tabelle 6.

| Aktivität | 8 h | 24 h | Aktivität | 8 h | 24 h |
|---|---|---|---|---|---|
| Puffer KPP 100 mM pH =7 1 mM $MgCl_2$ | 72 % | 70 % | Ethylacetat | 0 | 0 |
| 5 % Isopropanol | 77% | 77 % | Butylacetat | 74 % | 12 % |
| 10% Isopropanol | 86% | 85 % | Diethylether | 32 % | 20 % |
| 20 % Isopropanol | 100 % | 100 % | MTBE | 90 % | 81 % |
| 30 % Isopropanol | 0 % | 0 | Diisopropylether | 94% | 77 % |
| 5 % EtOH | 64 % | 65 % | Chloroform | 6 % | 0 % |
| 10% EtOH | 68 % | 70 % | Hexan | 115 % | 100 % |
| 20 % EtOH | 83 % | 80 % | Heptan | 113 % | 100 % |
| 30 % EtOH | 77 % | 75 % | Cyclohexan | 113 % | 100 % |
| 10 % DSMO | 80 % | 80 % | | | |
| 20 % DSMO | 79 % | 80 % | | | |

Wie aus Tabelle 6 ersichtlich zeigt die Oxidoreduktase aus L. reuteri eine erstaunliche gute Stabilität gegenüber organischen Lösungsmitteln. Ferner wird das Enzym in organischen wassermischbaren, sowie wasserunmischbaren Lösungsmitteln sogar stabilisiert im Vergleich mit der Inkubation im reinen Puffer.

5.7 Ermittlung von $K_m$ und $v_{max}$ für NADPH und Ethyl-2-oxopentanoate

Zur Bestimmung der $K_m$ und $v_{max}$ Werte von NADPH und Ethyl-2-oxopentanoat wurden folgende Ansätze gewählt:

A. Variation des NADPH/ Substratkonzentration konstant

970 µl Ethyl-2-oxopentanoat-Lösung in Kaliumphosphatpuffer pH =7,0
20 µl NADPH ( Konzentrationen von 200-5 µM Endkonzentration)
10 µl Enzymlösung (1:300)

B. Variation der Substratkonzentration/NADPH konstant

970 µl Ethyl-2-oxopentanoat-Lösung in Kaliumphosphatpuffer pH =7,0 (Konzentrationen von 10 bis 0,1 mM)
20 µl NADPH (0,2 mM Endkonzentration)
10 µl Enzymlösung (1:300)

| | Km | Vmax | |
|---|---|---|---|
| NADPH | 0,004 ± 0,0018 mM | 3080 U/ml | 18 500 U/g E.coli Feuchtgewicht |
| Ethyl 2-Oxopentanoat | 0,18± 0,07 mM | 3080 U/ml | 18 500 U/g E.coli Feuchtgewicht |

Als Enzymlösung wurde das aus 0,1 g rekombinanten E. coli Zellen gewonnene Lysat (600 µl) 1: 300 verdünnt eingesetzt.

5.8 Präperative Umsetzungen von Ethyl-2-oxo-4-phenylbutyrat

A. Coenzymregenerierung mit sekundärer Alkoholdehydrogenase aus Thermoanerobium brockii

Für einen präperativen Ansatz wurde ein Gemisch aus 4 ml Kaliumphosphatpuffer 100 mM, pH =7,0, 2 ml Isopropanol, 4 ml Ethyl-2-oxo-4-phenylbutyrat, 0,064 mg NADP, 1000 Units Oxidoreduktase aus L. reuteri und 10 mg ADH Thermoanerobium brockii (Fluka, etwa 100 U bezogen auf Oxidation von 2-Propanol) für 24 h bei Raumtemperatur unter ständiger Durchmischung inkubiert. Nach 24 h war das Substrat Ethyl-2-oxo-4-phenylbutyrat vollständig zu Ethyl-2-hydroxy-4-phenylbutyrat umgesetzt. Dabei wurde der entsprechende S-Alkohol zu 97 % gebildet.

B. Coenzymregenerierung mit sekundärer Alkohol-Dehydrogenase (KRED 1004, Biocatalytics Inc, Pasadena)

Für einen präperativen Ansatz wurde ein Gemisch aus 4 ml Kaliumphosphatpuffer 100 mM, pH =7,0, 2 ml Isopropanol, 4 ml Ethyl-2-oxo-4-phenylbutyrat, 0,064 mg NADP, 1000 Units Oxidoreduktase aus L. reuteri und 1 mg KRED 1004 (Biocatalytics Inc, Pasadena) für 2 h bei Raumtemperatur unter ständiger Durchmischung inkubiert. Nach 2 h war das Substrat Ethyl-2-oxo-4-phenylbutyrat vollständig zu Ethyl-2-hydroxy-4-phenylbutyrat umgesetzt. Dabei wurde der entsprechende S-Alkohol zu 98 % gebildet.

## Patentansprüche

1. Oxidoreduktase, welche 2-Oxosäureester in Gegenwart von NADPH und Wasser zu den entsprechenden S-2-hiydroxysäureestem reduziert, **dadurch gekennzeichnet, dass** sie mehr als 70 % Identität mit der Aminosäuresequenz SEQ ID NO: 18 und eine spezifische Aktivität von mehr als 1 μmol pro mg, bezogen auf die Umsetzung von Ethyl-2-oxo-4-phenyl-butyrat zu S-Ethyl-2-hydroxy-4-phenylbutyrat, aufweist.

2. Oxidoreduktase gemäß Anspruch 1, **dadurch gekennzeichnet, dass** sie 80% bis 99,5%, insbesondere 90% bis 99,5%, insbesondere bevorzugt 99% bis 99,5% Identität mit der Aminosäuresequenz SEQ ID NO: 18 aufweist.

3. Oxidoreduktase gemäß Anspruch 1 oder 2, **dadurch gekennzeichnet, dass** sie die Aminosäuresequenz SEQ ID NO: 18 aufweist.

4. Oxidoreduktase gemäß einem der Ansprüche 1 bis 3, **dadurch gekennzeichnet, dass** sie aus Lactobacillus (L.) reuteri, L. kefiri, L. kandleri, L. parabuchneri, L. cellobiosus oder L. fermentum erhältlich ist.

5. Oxidoreduktase gemäß einem der Ansprüche 1 bis 4, **dadurch gekennzeichnet, dass** sie 1 bis 50 Aminosäuren zusätzlich oder 1 bis 50 Aminosäuren weniger aufweiset als die Aminosäuresequenz SEQ ID NO: 18.

6. Oxidoreduktase gemäß Anspruch 5, **dadurch gekennzeichnet, dass** sie 1 bis 25 Aminosäuren, insbesondere 2 bis 20 Aminosäuren, bevorzugt 3 bis 10 Aminosäuren mehr oder weniger als Aminosäuresequenz SEQ ID NO: 18 aufweist.

7. Oxidoreduktase gemäß einem der Ansprüche 1 bis 6, **dadurch gekennzeichnet, dass** sie die Aminosäuresequenz SEQ ID NO: 18 aufweist und ein-, zwei-, drei-, vier- oder fünffach durch ein wasserlösliches Polymer modifiziert ist.

8. Oxidoreduktase gemäß Anspruch 7, **dadurch gekennzeichnet, dass** das wasserlösliche Polymer Polyethylenglycol ist.

9. Fusionsprotein, **dadurch gekennzeichnet, dass** es die Oxidoreduktase mit der Aminosäuresequenz SEQ ID NO: 18 darstellt und dass die Oxidoreduktase mit einem weiteren Polypeptid am N-terminalen oder Carboxy-terminalen Ende über eine Peptidbindung verbunden ist.

10. Antikörper, **dadurch gekennzeichnet**, das er spezifisch an die Oxidoreduktase gemäß SEQ ID NO: 18 bindet.

11. Isolierte Nukleinsäuresequenz, die für die Oxidoreduktase gemäß SEQ ID NO: 18 kodiert.

12. Isolierte DNA-Sequenz einer Oxidoreduktase gemäß einem der Ansprüche 1 bis 9,
wobei die DNA-Sequenz ausgewählt ist aus der Gruppe, bestehend aus

    a) SEQ ID NO: 19 oder ihrem komplementären Strang,
    b) einer DNA-Sequenz, welche mit SEQ ID NO: 19 oder ihrem komplementären Strang unter stringenten Bedingungen hybridisiert, und
    c) einer DNA-Sequenz, welche auf Grund der Degeneration des genetischen Codes ein Protein kodiert, das auch durch eine oder mehrere der DNA-Sequenzen gemäß a) oder b) kodiert ist.

13. Isolierte DNA-Sequenz, **dadurch gekennzeichnet, dass** sie mehr als 70 % Identität mit der DNA-Sequenz SEQ ID NO: 19 oder deren komplementären Strang aufweist und ein Protein kodiert, das eine spezifische Aktivität von mehr als 1 μmol pro mg, bezogen auf die Umsetzung von Ethyl-2-oxo-4-phenyl-butyrat zu S-Ethyl-2-hydroxy-4-phenylburytat, aufweist.

14. Isolierte DNA-Sequenz gemäß Anspruch 13, **dadurch gekennzeichnet, dass** sie 80% bis 99,5%, insbesondere 90% bis 99,5%, bevorzugt 99% bis 99,5% Identität mit der DNA-Sequenz SEQ ID NO: 19 aufweist.

**15.** Klonierungsvektor, **dadurch gekennzeichnet, dass** er eine oder mehrere der DNA-Sequenzen gemäß den Ansprüchen 11 bis 14 aufweist.

**16.** Expressionsvektor, **dadurch gekennzeichnet, dass** er eine oder mehrere der DNA-Sequenzen gemäß den Ansprüchen 11 bis 14 aufweist und in geeigneter Weise mit einer Expressionskontrollsequenz verbunden ist.

**17.** Wirtszelle, die eine Bakterien-, Hefe-, Insekten-, Pflanzen- oder Säugetierzelle ist und mit einem Expressionsvektor gemäß Anspruch 16 transformiert oder transfektiert wurde.

**18.** Verfahren zur enantioselektiven Gewinnung von S-2-Hydroxysäureester, **dadurch gekennzeichnet, dass** ein 2-Oxosäureester in Anwesenheit einer Oxidoreduktase gemäß einem der Ansprüche 1 bis 9, NADPH und Wasser zum entsprechenden S-2-Hydroxysäureester reduziert und der gebildete S-2-Hydroxysäureester isoliert wird.

**19.** Verfahren gemäß Anspruch 18, **dadurch gekennzeichnet, dass** als 2-Oxosäureester eine Verbindung der Formel I eingesetzt wird,

R2-C(O)-C(O)-O-R1 (I)

worin
R1 für

1. -($C_1$-$C_{20}$)-Alkyl, worin Alkyl geradkettig oder verzweigt ist,
2. -($C_2$-$C_{20}$)-Alkenyl, worin Alkenyl geradkettig oder verzweigt ist und je nach Kettenlänge ein, zwei, drei oder vier Doppelbindungen enthält,
3. -($C_2$-$C_{20}$)-Alkinyl, worin Alkinyl geradkettig oder verzweigt ist und gegebenenfalls ein, zwei, drei oder vier Dreifachbindungen enthält,
4. -($C_6$-$C_{14}$)-Aryl,
5. -($C_1$-$C_8$)-Alkyl-($C_6$-$C_{14}$)-Aryl,
6. -($C_5$-$C_{14}$)-Heterocyclus, der unsubstituiert oder ein- bis dreifach substituiert ist durch Halogen, Hydroxyl, Amino oder Nitro, oder
7. -($C_3$-$C_7$)-Cycloalkyl steht und

R2 für

1. -($C_1$-$C_{20}$)-Alkyl, worin Alkyl geradkettig oder verzweigt ist,
2. -($C_2$-$C_{20}$)-Alkenyl, worin Alkenyl geradkettig oder verzweigt ist und je nach Kettenlänge ein, zwei, drei oder vier Doppelbindungen enthält,
3. -($C_2$-$C_{20}$)-Alkinyl, worin Alkinyl geradkettig oder verzweigt ist und gegebenenfalls ein, zwei, drei oder vier Dreifachbindungen enthält,
4. -($C_6$-$C_{14}$)-Aryl,
5. -($C_1$-$C_8$)-Alkyl-($C_6$-$C_{14}$)-Aryl,
6. -($C_5$-$C_{14}$)-Heterocyclus, der unsubstituiert oder ein- bis dreifach substituiert ist durch Halogen, Hydroxyl, Amino oder Nitro, oder
7. -($C_3$-$C_7$)-Cycloalkyl steht,

wobei die oben unter 1. bis 7. genannten Reste unsubstituiert sind oder ein- bis dreifach substituiert sind unabhängig voneinander durch

a) -OH,
b) Halogen, wie Fluor, Chlor, Brom oder Jod,
c) -$NO_2$,
d) -C(O)-O-($C_1$-$C_{20}$)-Alkyl, worin Alkyl gerade oder verzweigt ist und unsubstituien oder ein- bis dreifach substituiert ist durch Halogen, Hydroxyl, Amino oder Nitro, oder
e) -($C_5$-$C_{14}$)-Heteroeyclus, der unsubstituiert oder ein- bis dreifach substituiert ist durch Halogen, Hydroxyl, Amino oder Nitro.

**20.** Verfahren zur enantioselektiven Gewinnung von S-2-Hydroxysäureester, **dadurch gekennzeichnet, dass**

a) ein 2-Oxosäureester in Anwesenheit von Oxidoreduktase gemäß einem der Ansprüche 1 bis 9, NADPH und Wasser zum entsprechenden S-2-Hydroxysäureester reduziert wird,
b) das durch die Oxidoreduktase gebildete NADP mit einer Dehydrogenase und einem Cosubstrat gleichzeitig zu NADPH reduziert wird, und
c) der gebildete chirale S-2-Hydroxysäureester isoliert wird.

**21.** Verfahren gemäß Anspruch 20, **dadurch gekennzeichnet, dass** als 2-Oxosäureester eine Verbindung der Formel I gemäß Anspruch 19 eingesetzt wird.

**22.** Verfahren gemäß Anspruch 20 oder 21, **dadurch gekennzeichnet, dass** als Dehydrogenase die Alkohol-Dehydrogenase aus Thermoanaerobium brockii, Lactobacillus kefir oder Lactobacillus brevis eingesetzt und als Cosubstrat Ethanol, 2-Propanol, 2-Butanol, 2-Pentanol oder 2-Octanol verwendet wird.

**23.** Verfahren gemäß Anspruch 20 oder 21, **dadurch gekennzeichnet, dass** als Dehydrogenase die Glucose-Dehydrogenase und als Cosubstrat Glucose eingesetzt oder die NADPH-abhängige Formiat-Dehydrogenase und als Cosubstrat ein Salz der Ameisensäure wie Ammoniumformiat, Natriumformiat oder Calziumformiat eingesetzt werden.

**24.** Verfahren gemäß einem der Ansprüche 20 bis 23, **dadurch gekennzeichnet, dass** die Reaktionen in Anwesenheit eines organischen Lösungsmittels durchführt werden.

**25.** Verfahren gemäß Anspruch 24, **dadurch gekennzeichnet, dass** als organisches Lösungsmittel Diethylether, tertiär-Butylmethylether, Diisopropylether, Dibutylether, Butylacetat, Heptan, Hexan oder Cyclohexan eingesetzt wird.

**26.** Verfahren gemäß Anspruch 24 oder 25, **dadurch gekennzeichnet, dass** die organische Phase 5% bis 80% des gesamten Reaktionsvolumens, bevorzugt 10% bis 40%, beträgt.

## Claims

**1.** An oxidoreductase, which reduces 2-oxo acid esters in the presence of NADPH and water to the corresponding S-2-hydroxy acid esters, **characterized in that** it has more than 70% identity with the amino acid sequence SEQ ID NO:18 and has a specific activity of more than 1 μmol per mg, based on the reaction of ethyl-2-oxo-4-phenyl butyrate to S-ethyl-2-hydroxy-4-phenyl butyrate.

**2.** An oxidoreductase according to claim 1, **characterized in that** it has 80% to 99.5%, particularly 90% to 99.5% and in particular preferably 99% to 99.5% identity with the amino acid sequence SEQ ID NO:18.

**3.** An oxidoreductase according to claim 1 or 2, **characterized in that** it has the SEQ ID NO:18.

**4.** An oxidoreductase according to any of claims 1 to 3, **characterized in that** it is obtainable from Lactobacillus (L.) reuteri, L. kefir, L. kandleri, L. parabuchneri, L. cellobiosus or L. fermentum.

**5.** An oxidoreductase according to any of claims 1 to 4, **characterized in that** it has 1 to 50 amino acids more or 1 to 50 amino acids less than the amino acid sequence SEQ ID NO:18.

**6.** An oxidoreductase according to claim 5, **characterized in that** it has 1 to 25 amino acids, especially 2 to 20 amino acids, preferably 3 to 10 amino acids more or less than the amino acid sequence SEQ ID NO:18.

**7.** An oxidoreductase according to any of claims I to 6, **characterized in that** it has the amino acid sequence SEQ ID NO:18 and is modified once, twice, three, four or five times by a water-soluble polymer.

**8.** An oxidoreductase according to claim 7, **characterized in that** the water-soluble polymer is polyethylene glycol.

**9.** A fusion protein, **characterized in that** it constitutes the oxidoreductase with the amino acid sequence SEQ ID NO: 18 and that the oxidoreductase is linked to another polypeptide at the N-terminal or carboxy-terminal end via a peptide bond.

**10.** An antibody, **characterized in that** it binds specifically to the oxidoreductase according to SEQ ID NO:18.

**11.** An isolated nucleic acid sequence coding for the oxidoreductase according to SEQ ID NO:18.

**12.** An isolated DNA sequence of an oxidoreductase according to any of claims 1 to 9, wherein the DNA sequence is selected from the group consisting of

a) SEQ ID NO:19 or the complementary strand thereof,
b) a DNA sequence which hybridizes with SEQ ID NO:19 or the complementary strand thereof under stringent conditions, and
c) a DNA sequence encoding, due to the degeneration of the genetic code, a protein which is also encoded by one or several of the DNA sequences according to a) or b).

**13.** An isolated DNA sequence, **characterized in that** it has more than 70% identity with the DNA sequence SEQ ID NO:19 or the complementary strand thereof and encodes a protein having a specific activity of more than 1 μmol per mg, based on the reaction of ethyl-2-oxo-4-phenyl butyrate to S-ethyl-2-hydroxy-4-phenyl butyrate.

**14.** An isolated DNA sequence according to claim 13, **characterized in that** it has 80% to 99.5%, especially 90% to 99,5%, preferably 99% to 99,5% identity with the DNA sequence SEQ ID NO:19.

**15.** A cloning vector, **characterized in that** it has one or several of the DNA sequences according to claims 11 to 14.

**16.** An expression vector, **characterized in that** it has one or several of the DNA sequences according to claims 11 to 14 and is appropriately linked to an expression control sequence.

**17.** A host cell, which is a bacterial, yeast, insect, plant or mammalian cell and has been transformed or transfected with an expression vector according to claim 16.

**18.** A method for the enantioselective production of S-2-hydroxy acid ester, **characterized in that** a 2-oxo acid ester is reduced in the presence of an oxidoreductase according to any of claims 1 to 9, NADPH and water to the corresponding S-2-hydroxy acid ester and that the S-2-hydroxy acid ester formed is isolated.

**19.** Method according to claim 18, **characterized in that** a compound of the formula I,

R2-C(O)-C(O)-O-R1 (I),

wherein
R1 stands for

1. -($C_1$-$C_{20}$)-alkyl, which is linear-chain or branched,
2. -($C_2$-$C_{20}$)-alkenyl, which is linear-chain or branched and contains one, two, three or four double bonds, depending on the chain length,
3. -($C_2$-$C_{20}$)-alkynyl, which is linear-chain or branched and optionally contains one, two, three or four triple bonds,
4. -($C_6$-$C_{14}$)-aryl,
5. -($C_1$-$C_8$)-alkyl-($C_6$-$C_{14}$)-aryl,
6. -($C_5$-$C_{14}$)-heterocycle, which is unsubstituted or substituted one to three times by halogen, hydroxyl, amino or nitro, or
7. -($C_3$-$C_7$)-cycloalkyl, and

R2 stands for

1. -($C_1$-$C_{20}$)-alkyl, which is linear-chain or branched,
2. -($C_2$-$C_{20}$)-alkenyl, which is linear-chain or branched and contains one, two, three or four double bonds, depending on the chain length,
3. -($C_2$-$C_{20}$)-alkynyl, which is linear-chain or branched and optionally contains one, two, three or four triple bonds,
4. -($C_6$-$C_{14}$)-aryl,

5. $(C_1-C_8)$-alkyl-$(C_6-C_{14})$-aryl,
6. -$(C_5-C_{14})$-heterocycle, which is unsubstituted or substituted one to three times by halogen, hydroxyl, amino or nitro, or
7. -$(C_3-C_7)$-cycloalkyl,
wherein the moieties mentioned above under 1 to 7 are unsubstituted or substituted one to three times, independently of each other, by

a) -OH,
b) halogen such as fluorine, chlorine, bromine or iodine,
c) -$NO_2$,
d) -C(O)-O-$(C_1-C_{20})$-alkyl, wherein alkyl is linear-chain or branched and unsubstituted or substituted one to three times by halogen, hydroxyl, amino or nitro, or
e) -$(C_5-C_{14})$-heterocycle, which is unsubstituted or substituted one to

three times by halogen, hydroxyl, amino or nitro,

is used as the 2-oxo acid ester.

**20.** A method for the enantioselective production of S-2-hydroxy acid ester, **characterized in that**

a) a 2-oxo acid ester is reduced to the corresponding S-2-hydroxy acid ester in the presence of oxidoreductase according to any of claims 1 to 9, NADPH and water,
b) the NADP formed by the oxidoreductase is simultaneously reduced to NADPH with a dehydrogenase and a co-substrate, and
c) the chiral S-2-hydroxy acid ester formed is isolated.

**21.** A method according to claim 20, **characterized in that** a compound of formula I according to claim 19 is used as the 2-oxo acid ester.

**22.** A method according to claim 20 or 21, **characterized in that** the alcohol dehydrogenase from Thermoanaerobium brockii, Lactobacillus kefir or Lactobacillus brevis is used as the dehydrogenase and ethanol, 2-propanol, 2-butanol, 2-pentanol or 2-octanol as the co-substrate.

**23.** A method according to claim 20 or 21, **characterized in that** the glucose dehydrogenase is used as the dehydrogenase and glucose as the co-substrate or the NADPH-dependent formate dehydrogenase is used and a salt of formic acid such as ammonium formate, sodium formate or calcium formate as the co-substrate.

**24.** A method according to any of claims 20 to 23, **characterized in that** the reactions are carried out in the presence of an organic solvent.

**25.** A method according to claim 24, **characterized in that** diethyl ether, tertiary butyl methyl ether, diisopropyl ether, dibutyl ether, butyl acetate, heptane, hexane or cyclohexane is used as the organic solvent.

**26.** A method according to claim 24 or 25, **characterized in that** the organic phase is 5% to 80% of the entire reaction volume, preferably 10% to 40%.

## Revendications

**1.** Oxydoréductase qui réduit des esters d'acide 2-oxo en présente de NADPH et d'eau en esters d'acide S-2-hydroxylique correspondants,
**caractérisée en ce que**
elle présente plus de 70 % d'identité avec la séquence d'acides aminés SEQ ID NO : 18 et une activité spécifique de plus de 1 μmole par mg en termes de conversion du butyrate d'éthyl-2-oxo-4-phényle en butyrate de S-éthyl-2-hydroxy-4-phényle.

**2.** Oxydoréductase selon la revendication 1, **caractérisée en ce qu'**elle présente de 80 % à 99,5 %, en particulier de 90 % 99,5 % et de façon particulièrement préférable de 99 % à 99,5 % d'identité avec la séquence d'acides aminés SEQ ID NO : 18.

3. Oxydoréductase selon les revendications 1 ou 2, **caractérisé en ce qu'**elle présente la séquence d'acides aminés SEQ ID NO : 18.

4. Oxydoréductase selon l'une des revendications 1 à 3, **caractérisée en ce qu'**elle peut être obtenue à partir de lacrobacillus (L.) reuteri, L. kefiri, L. kandleri, L. parabuchneri, L. cellobiosus ou L. fermentum.

5. Oxydoréductase selon l'une des revendications 1 à 4, **caractérisée en ce qu'**elle présente 1 à 50 acides aminés de plus ou 1 à 50 acides aminés de moins que la séquence d'acides aminés SEQ ID NO : 18.

6. Oxydroréductase selon la revendication 5, **caractérisée en ce qu'**elle présente 1 à 25 acides aminés, en particulier 2 à 20 acides aminés et de préférence 3 à 10 acides aminés de plus ou de moins que la séquence d'acides aminés SEQ ID NO : 18.

7. Oxydoréductase selon l'une des revendications 1 à 6, **caractérisée en ce qu'**elle présence la séquence d'acides aminés SEQ ID NO : 18 et est modifiée une, deux, trois, quatre ou cinq fois par un polymère soluble dans l'eau.

8. Oxydoréductase selon la revendication 7, **caractérisée en ce que** le polymère soluble dans l'eau est le polyéthylène glycol.

9. Protéine de fusion, **caractérisée en ce qu'**elle représente une oxydoréductase qui présente la séquence d'acides aminés SEQ ID NO : 18 et **en ce que** l'oxydoréductase est liée à un autre polypeptide à son extrémité N-terminale ou à son extrémité carboxy-terminale par l'intermédiaire d'une liaison peptidique.

10. Anticorps **caractérisé en ce qu'**il se lie spécifiquement à l'oxydoréductase selon la SEQ ID NO : 18.

11. Séquence isolée d'acides nucléiques qui code pour l'oxydoréductase selon la SEQ ID NO : 18.

12. Séquence d'ADN isolée d'une oxydoréductase selon l'une des revendications 1 à 9, la séquence d'ADN étant sélectionnée dans l'ensemble constitué de :

    a) la SEQ ID NO : 19 ou son brin complémentaire,
    b) une séquence d'ADN qui s'hybride avec la SEQ ID NO : 19 ou son brin complémentaire dans des conditions stringentes et
    c) une séquence d'ADN qui, sur base de la dégénérescence du code génétique, code pour une protéine qui est également codée par une ou plusieurs des séquences d'ADN selon a) ou b).

13. Séquence d'ADN isolée **caractérisée en ce qu'**elle présente plus de 70 % d'identité avec la séquence d'ADN SEQ ID NO : 19 ou son brin complémentaire et **en ce qu'**elle code pour une protéine qui présente une activité spécifique supérieure à 1 μmole par mg en termes de conversion du butyrate d'éthyl-2-oxo-4-phényle en butyrate de S-éthyl-2-hydroxy-4-phényle.

14. Séquence d'ADN isolée selon la revendication 13, **caractérisée en ce qu'**elle présente de 80 % à 99,5 %, en particulier de 90 % à 99,5 % et de préférence de 99 % à 99,5 % d'identité avec la séquence d'ADN SEQ ID NO : 19.

15. Vecteur de clonage **caractérisé en ce qu'**il présente une ou plusieurs des séquences d'ADN selon les revendications 11 à 14.

16. Vecteur d'expression **caractérisé en ce qu'**il présente une ou plusieurs des séquences d'ADN selon les revendications 11 à 14 et est relié de manière appropriée à une séquence de contrôle d'expression.

17. Cellule-hôte qui est une cellule de bactérie, de levure, d'insecte, de plante ou de mammifère, et transformée ou transfectée par un vecteur d'expression selon la revendication 16.

18. Procédé d'obtention énantiosélective d'esters d'acide S-2-hydroxylique, **caractérisé en ce qu'**un ester d'acide 2-oxo est réduit en présence d'une oxydoréductase selon l'une des revendications 1 à 9, de NADPH et d'eau en ester correspondant d'acide S-2-hydroxylique et **en ce que** l'ester d'acide S-2-hydroxylique formé est isolé.

**19.** Procédé selon la revendication 18, **caractérisé en ce que** comme ester d'acide 2-oxo, il utilise un composé de formule I :

R2-C(O)-C(O)-O-R1 (1)

dans laquelle
R1 représente :

1. un alkyle en $C_1$ à $C_{20}$, l'alkyle étant à chaîne linéaire ou ramifiée,
2. un alcényle en $C_2$ à $C_{20}$, l'alcényle étant à chaîne linéaire ou ramifiée et contenant deux, trois ou quatre doubles liaisons selon la longueur de la chaîne,
3. un alcinyle en $C_2$ à $C_{20}$, l'alcinyle étant à chaîne linéaire ou ramifiée et contenant éventuellement une, deux, trois ou quatre triples liaisons,
4. un aryle en $C_6$ à $C_{14}$,
5. un (alkyle en $C_1$ à $C_8$)-(aryle en $C_6$ à $C_{14}$),
6. un hétérocycle en $C_5$ à $C_{14}$ non substitué ou substitué de une à trois fois avec un halogène, un hydroxyle, un amino ou un nitro ou
7. un cycloalkyle en $C_3$ à $C_7$ et

R2 représente :

1. un alkyle en $C_1$ à $C_{20}$, l'alkyle étant à chaîne linéaire ou ramifiée,
2. un alcényle en $C_2$ à $C_{20}$, l'alcényle étant à chaîne linéaire ou ramifiée et contenant deux, trois ou quatre doubles liaisons selon la longueur de la chaîne,
3. un alcinyle en $C_2$ à $C_{20}$, l'alcinyle étant à chaîne linéaire ou ramifiée et contenant éventuellement une, deux, trois ou quatre triples liaisons,
4. un aryle en $C_6$ à $C_{14}$,
5. un (alkyle en $C_1$ à $C_8$)-(aryle en $C_6$ à $C_{14}$),
6. un hétérocycle en $C_5$ à $C_{14}$ non substitué ou substitué de une à trois fois avec un halogène, un hydroxyle, un amino ou un nitro ou
7. un cycloalkyle en $C_3$ à $C_7$ et

les groupes mentionnés plus haut aux points 1. à 7, sont non substitués ou sont substitués de une à trois fois, indépendamment les uns des autres, avec :

a) -OH,
b) un halogène tel que le fluor, le chlore, le brome ou l'iode,
c) $-NO_2$,
d) un -C(O)-O-(alkyle en $C_1$ à $C_{20}$), l'alkyle étant linéaire ou ramifié, non substitué ou substitué de une à trois fois avec un halogéné, un hydroxyle, un amino ou un nitro ou
e) un hétérocycle en $C_5$ à $C_{14}$ non substitué ou substitue de une à trois fois avec un halogène, un hydroxyle, un amino ou un nitro.

**20.** Procédé d'obtention énantio-sélective d'esters d'acide S-2-hydroxylique,
**caractérisé en ce que**

a) un ester d'acide 2-oxo est réduit en présence d'une oxydoréductase selon l'une des revendications 1 à 9, de NADPH et d'eau en ester correspondant d'acide S-2-hydroxylique,
b) le NADP formé par l'oxydoréductase est réduit en même temps en NADPH par une déshydrogénase et un co-substrat et
c) l'ester chiral d'acide S-2-hydroxylique ainsi formé est isolé.

**21.** Procédé selon la revendication 20, **caractérisé en ce qu'**on utilise comme ester d'acide 2-oxo un composé de formule I selon la revendication 19.

**22.** Procédé selon les revendications 20 ou 21, **caractérisé en ce que** comme déshydrogénase, on utilise une déshydrogénase d'alcool produite par Thermoanaérobium brockii, Lactobacillus kefir ou hactobacillus brevis et comme co-substrat l'éthanol, le 2-propanol, le 2-butanol, le 2-penranol ou le 2-octanol.

**23.** Procède selon les revendications 20 ou 21, **caractérisé en ce que** comme déshydrogénase, on utilise une glucose-déshydrogénase et comme co-substrat le glucose ou une fermiate-déshydrogénase dépendant du NADPH et comme co-substrat un sel d'acide formique tel que le formate d'ammonium, le formiate de sodium ou le formiate de calcium.

**24.** Procédé selon l'une des revendications 20 à 23, **caractérisé en ce que** les réactions sont conduites en présence d'un solvant organique.

**25.** Procédé selon la revendication 24, **caractérisé en ce qu'**on utilise comme solvant organique l'éther de diéthyle, un éther tertiaire de butyle et de méthyle, l'éther de diisopropyle, l'éther de dibutyle, l'acétate de butyle, l'heptane, l'hexane ou le cyclohexane.

**26.** Procédé selon les revendications 24 ou 25, **caractérisé en ce que** la phase organique représente de 5 % à 80 % et de préférence de 10 % à 40 % de l'ensemble du volume de réaction.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5686275 A
- US 6033882 A
- DE 19610984
- EP 0456107 A
- WO 9732012 A
- DE 10119274


### In der Beschreibung aufgeführte Nicht-Patentliteratur


- **K. Faber.** Biotransformations in Organic Chemistry, A Textbook. Springer, 2000
- **Kalaritis, P.** Kinetic Resolution of 2-substituted Esters catalysed by a Lipase Ex. Pseudomonas floureszens. *J. Org. Chem.,* 1991, vol. 55, 812-815
- **Paola D'Arrigo ; Giuseppe Pedrocchi-Fantoni ; Stefano Servi.** Stereoselective Synthesis of Chiral Compounds Using Whole- Cell Biocatalysis. *Ramesh N. Patel Stereoselective Biocatalyse,* 2000, 14
- *Eur J Biochem,* 1994, vol. 222 (3), 1025-32
- **Tamura Y. et al.** Two forms of NAD-dependent D-mandelate dehydrogenase in Enterococcus faecalis IAM 10071. *Appl Environ Microbiol,* Februar 2002, vol. 68 (2), 947-51
- Alcohol Dehydrogenases-Characteristics, Design of Reaction Conditions. **J. Peters.** Biotechnology , Biotransformations I (Rehm and Reed) 9. WILEY-VCH -Verlag, 1998
- **Liese, K. Seelbach ; C.Wandrey.** Industrial Biotransformations. WILEY-VCH -Verlag, 2000
- **Sambrok ; Russel.** Molecular Cloning a laboratory Manual. vol. 1
- **Tishkov et al.** *J. Biotechnol. Bioeng.,* 1999, vol. 64, 187-193
- **Lowry et al.** *Journal of Biological Chemistry,* 1951, vol. 193, 265-275
- **Peterson et al.** *Analytical Biochemistry,* 1979, vol. 100, 201-220

SEQUENCE LISTING

<110> Juelich Enzyme Products GmbH
<120> Oxidoreduktase aus Lactobacillus reuteri
<130> (TH)4061
<160> 19
<170> PatentIn version 3.1

<210> 1
<211> 15
<212> PRT
<213> Lactobacillus reuteri<400> 1

Met Lys Asn Ile Met Ile Ala Gly Ala Gly Val Leu Gly Ser Gln
1 5 10 15

<210> 2
<211> 12
<212> PRT
<213> Lactobacillus reuteri
<220>
<221> PEPTIDE
<222> (1)..(12)
<223>

<400> 2

Ser Asp Tyr Glu Arg Asp Leu His Leu Thr Asp Lys
1 5 10

<210> 3

<211> 26
<212> DNA
<213> artificial
<400> 3
atgaaraaya tyatgatygc hggcgc 26

<210> 4
<211> 26
<212> DNA
<213> artificial
<400> 4
atgaaraaya tyatgatygc hggtgc 26

<210> 5
<211> 21
<212> DNA
<213> artificial
<400> 5
rtgharatcm cgttcrtaat c 21

<210> 6
<211> 21
<212> DNA
<213> artificial
<400> 6
rtgharatcm cgttcrtagt c 21

<210> 7
<211> 159
<212> DNA
<213> Lactobacillus reuteri
<400> 7

atgaagaata tcatgattgc tggtgctggc gtattaggta gtcagattgc atatcaaacg 60

gctttatccg gctttaatgt cagcgtatat aatcaccata ttgataccgc tgagcgacgg 120

attaaggcgc tgaaaagtga ttacgaacgt gatttacat 159

<210> 8
<211> 21
<212> DNA
<213> artificial
<400> 8
atccggcttt aatgtcagcg t 21

<210> 9
<211> 23
<212> DNA
<213> artificial
<400> 9
cgacggatta aggcgctgaa aag 23

<210> 10
<211> 22
<212> DNA

<213> artificial
<400> 10
ctacctaata cgccagcacc ag 22

<210> 11
<211> 18
<212> DNA
<213> Artificial
<400> 11
gccagcacca gcaatcat 18

<210> 12
<211> 1257
<212> DNA
<213> Lactobacillus reuteri
<400> 12

cgacggatta aggcgctgaa aagtgattat gaacgtgact tgcatttgac tgataaggaa 60

tttcaacagg gccttaataa tattaaagtg attactgatg atgttgcgac cgcggttaaa 120

gatgctgatt taatgattga agcattacca gaatcgttag agttaaagga gcagttttac 180

gaagaggttt cagaattagc ccctgaaaaa acaatctttg ctagcaactc ctctacattt 240

atccctagcc aactagctcc ttatactgat cggccagaaa aatttcttaa tatgcacttt 300

gctaaccaaa tttggaaatt taatgtggtc gaaattatgg gcacctccca aacaagtcca 360

gaggtgattg aagaagctac aaagtttgcc cgggaaataa agatggttcc tgttatcctt 420

aataaagaac aacacggtta tattctgaat tcatggtgaa tcaggaatgc ctggttatga 480

ctttggtgac gattatggta agcctcttga taagactgct gttgaagcac ttcatgacgc 540

aatcatggct gaagatgaag tcatcttggt tccaactggt tcatacacta acattgcatt 600

actctttagc gaatacccag aagtaaagag tcacattaag caaatcgttg cgatgggtgg 660

ttcattctct ggcggtaaca tgaccagtgt agctgaattt aacgtcttca ctgatccgga 720

tgctgctaag attatgtaca atgcgggtgt tccaattgta actgttggat tggatgttac 780

cttaaaggcg ctcttaactg cggatacgat tgaaaaactt ggtagtctta ataagactgg 840

tgaaatgcta catggattaa tcacgcacta taatgatggt agtgatcaag ggcgtccaat 900

gcacgatgtt aatactatct tctaccttct tcatccagaa gcatttacta cgaaggacat 960

gtgggttgat gttcaaacag acggtccagc aatcggtgct actgttggtg acattcgcgc 1020

tgcttaccat gatggcaaga ccaacgcaaa agtttgttta gatattgatg ctgaatactt 1080

caataagtgg ttcttagaag aagtaagcaa aatgaaataa gatactaaga gctgagagtt 1140

tgctttcggc tctttttatt actttctagt gtaagcgctt cacgatataa tagtaatagt 1200

taaagtaagg aggaaatacc aaatgaagaa tataatgatt gctggtgctg gcaagcc 1257

<210> 13
<211> 23
<212> DNA
<213> Artificial
<400> 13
ccagaggtga ttgaagaagc tac 23

<210> 14
<211> 19
<212> DNA
<213> Artificial
<400> 14
ccgggaaata aagatggtt 19

<210> 15
<211> 822
<212> DNA
<213> Lactobacillus reuteri
<400> 15

ccgggaaata aagatggttc ctgttatcct taataaagaa caacacggtt atattctgaa 60

ttcccttttg attccgttac ttgcatctgg tcttagttta tgggctaaag gagttgctga 120

tccagcgatg attgataagg attggatgat ttcgacaggt gcaccaatgg gaccatttgg 180

tattttagat atggttggtt tacggacagc agcgcaaatc gaacggaatg cctatgccca 240

gactaaggat gaaagtcata aggaaattgc cgataagatg gaacagatga ttcaagaagg 300

gcacgaagga aaagaatcgg gacaaggatt ttataattat ccgaatccag ccttcatgga 360

tccagatttt ctgaagcatt aagattaagt attgaaaacg ttaggaaagg gagcaagata 420

agagtcgctc gtgactgtta tctcactccc ttattttgcg ctccaatttt cacgtaatcc 480

ggttgcaaca atggcaaaat tacggtacag tagtagagaa cgtgaaacaa aaaatgtttc 540
acgaaaggag acctaataaa tgaatcctga acaatttcaa caagcattag ctgatcatgg 600
tattacctta tcagcagaac aaatgcaaca atttgctgac tattaccaat tattagttga 660
aactaatgaa catggtgggt tgatgttcaa cagacggtcc agcatcggtg ctactgtggt 720
gacattcgcg ctgcttacat gatggcagac caacgcaaag tttgttagat atgatgctga 780
atacttcaat aagtggttct taaagaagta gcaaatgaat ag 822

<210> 16
<211> 33
<212> DNA
<213> Artificial
<400> 16
gcggaattcc atatgaagaa tatcatgatt gct 33

<210> 17
<211> 28
<212> DNA
<213> Artificial
<400> 17
cccaagctta atgcttcaga aaatctgg 28

<210> 18
<211> 294
<212> PRT
<213> Lactobacillus reuteri
<400> 18

Met Lys Asn Ile Met Ile Ala Gly Ala Gly Val Leu Gly Ser Gln Ile
1 5 10 15

Ala Tyr Gln Thr Ala Leu Ser Gly Phe Asn Val Ser Val Tyr Asn His
20 25 30

His Ile Asp Thr Ala Glu Arg Arg Ile Lys Ala Leu Lys Ser Asp Tyr
35 40 45

Glu Arg Asp Leu His Leu Thr Asp Lys Glu Phe Gln Gln Gly Leu Asn
50 55 60

Asn Ile Lys Val Ile Thr Asp Asp Val Ala Thr Ala Val Lys Asp Ala
65 70 75 80

Asp Leu Met Ile Glu Ala Leu Pro Glu Ser Leu Glu Leu Lys Glu Gln
85 90 95

Phe Tyr Glu Glu Val Ser Glu Leu Ala Pro Glu Lys Thr Ile Phe Ala
100 105 110

Ser Asn Ser Ser Thr Phe Ile Pro Ser Gln Leu Ala Pro Tyr Thr Asp
115 120 125

Arg Pro Glu Lys Phe Leu Asn Met His Phe Ala Asn Gln Ile Trp Lys
130 135 140

Phe Asn Val Val Glu Ile Met Gly Thr Ser Gln Thr Ser Pro Glu Val
145 150 155 160

Ile Glu Glu Ala Thr Lys Phe Ala Arg Glu Ile Lys Met Val Pro Val
165 170 175

Ile Leu Asn Lys Glu Gln His Gly Tyr Ile Leu Asn Ser Leu Leu Ile
180 185 190

Pro Leu Leu Ala Ser Gly Leu Ser Leu Trp Ala Lys Gly Val Ala Asp
195 200 205

Pro Ala Met Ile Asp Lys Asp Trp Met Ile Ser Thr Gly Ala Pro Met
210 215 220

Gly Pro Phe Gly Ile Leu Asp Met Val Gly Leu Arg Thr Ala Ala Gln
225 230 235 240

Ile Glu Arg Asn Ala Tyr Ala Gln Thr Lys Asp Glu Ser His Lys Glu
245 250 255

Ile Ala Asp Lys Met Glu Gln Met Ile Gln Glu Gly His Glu Gly Lys
260 265 270

Glu Ser Gly Gln Gly Phe Tyr Asn Tyr Pro Asn Pro Ala Phe Met Asp
275 280 285

Pro Asp Phe Leu Lys His
290

<210> 19
<211> 885
<212> DNA
<213> Lactobacillus reuteri
<400> 19

atgaagaata tcatgattgc tggtgctggc gtattaggta gtcagattgc atatcaaacg 60

gctttatccg gctttaatgt cagcgtatat aatcaccata ttgataccgc tgagcgacgg 120

attaaggcgc tgaaaagtga ttatgaacgt gacttgcatt tgactgataa ggaatttcaa 180

cagggcctta ataatattaa agtgattact gatgatgttg cgaccgcggt taaagatgct 240

gatttaatga ttgaagcatt accagaatcg ttagagttaa aggagcagtt ttacgaagag 300

gtttcagaat tagcccctga aaaaacaatc tttgctagca actcctctac atttatccct 360

agccaactag ctccttatac tgatcggcca gaaaaatttc ttaatatgca ctttgctaac 420

caaatttgga aatttaatgt ggtcgaaatt atgggcacct cccaaacaag tccagaggtg 480

attgaagaag ctacaaagtt tgcccgggaa ataaagatgg ttcctgttat ccttaataaa 540

gaacaacacg gttatattct gaattccctt ttgattccgt tacttgcatc tggtcttagt 600

ttatgggcta aaggagttgc tgatccagcg atgattgata aggattggat gatttcgaca 660

ggtgcaccaa tgggaccatt tggtatttta gatatggttg gtttacggac agcagcgcaa 720

atcgaacgga atgcctatgc ccagactaag gatgaaagtc ataaggaaat tgccgataag 780

atggaacaga tgattcaaga agggcacgaa ggaaaagaat cgggacaagg attttataat 840

tatccgaatc cagccttcat ggatccagat tttctgaagc attaa 885